# EUROPEAN PATENT SPECIFICATION

(11) **EP 4 176 800 B1**
(45) Date of publication and mention of the grant of the patent: **10.06.2026**
(21) Application number: 22198169.9
(22) Date of filing: 17.08.2016
(51) Int. Cl.: A61B 5/00, A61B 7/00, A61B 5/08

(54) **SCREENER FOR SLEEP DISORDERED BREATHING**
SCREENER FÜR SCHLAFATMUNGSSTÖRUNGEN
CRIBLE POUR TROUBLES RESPIRATOIRES DU SOMMEIL

(30) Priority: 17.08.2015 US 201562205823 P
(43) Date of publication of application: 10.05.2023
(62) Divisional of application: 16767143.7
(73) Proprietor: ResMed Sensor Technologies Limited, Sandyford, Dublin D18 T6T7 (IE)
(72) Inventor: MCMAHON, Stephen, Dundrum Dublin 14 (IE)
(74) Representative: Vossius & Partner Patentanwälte Rechtsanwälte mbB

(56) References cited:
- EP-A1- 2 457 504
- WO-A1-2012/155257
- US-A1- 2011 190 651

## Description

This application claims the benefit of the filing date of United States Provisional Patent Application No. 62/205,823, filed on 17 August 2015.

### 1 BACKGROUND OF THE TECHNOLOGY

### 1.1 FIELD OF THE TECHNOLOGY

The present technology relates to one or more of the detection, diagnosis and prevention of respiratory-related conditions. The present technology also relates to medical apparatus, devices, systems, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the air into the venous blood and carbon dioxide to move out. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2011.

A range of respiratory disorders exist. Certain disorders may be characterised by particular events, for example, snoring, apneas, hypopneas, hyperpneas, etc. Some of these respiratory disorders may be referred to as Sleep Disorder Breathing (SDB).

Obstructive Sleep Apnea (OSA) is characterized by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. *See* US Patent No. 4,944,310 (Sullivan).

Cheyne-Stokes Respiration (CSR) is a disorder of a patient's respiratory controller, in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. *See* US Patent No. 6,532,959 (Berthon-Jones).

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

### 1.2.2 Treatment Systems and Devices

Once diagnosed, a range of therapies have been used to treat or ameliorate the above conditions. These therapies may be provided by a system or device that comprises, for example, a Respiratory Pressure Therapy Device (RPT device), an air circuit, a humidifier, a patient interface, and a data management device.

Continuous Positive Airway Pressure (CPAP) therapy, for example, has been used to treat Obstructive Sleep Apnea (OSA). The hypothesis is that continuous positive airway pressure acts as a pneumatic splint and may prevent upper airway occlusion by pushing the soft palate and tongue forward and away from the posterior oropharyngeal wall. Non-invasive ventilation (NIV), as another example, provides ventilatory support to a patient through the upper airways to assist the patient in taking a full breath and/or maintain adequate oxygen levels in the body by doing some or all of the work of breathing. The ventilatory support is provided via a patient interface, such as a mask. NIV has been used to treat CSR, OHS, COPD, MD and Chest Wall disorders. In severe cases, invasive ventilation (IV) provides ventilatory support to patients that are no longer able to effectively breathe themselves and may be provided using a tracheostomy tube.

In addition to their therapeutic benefits, it may be advantageous for otherwise healthy individuals to take advantage of such therapies to prevent respiratory disorders from arising. Treatment of a potential case of OSA by CPAP therapy, for example, may be a voluntary treatment. Diagnosis is, of course, required before any such preventative treatments can be initiated.

### 1.2.3 Diagnosis and Monitoring Systems

Polysomnography (PSG) is a conventional system for diagnosis and prognosis of cardio-pulmonary disorders such as the conditions described above. PSG typically involves the placement of 15 to 20 contact sensors on a person in order to record various bodily signals such as electroencephalography (EEG), electrocardiography (ECG), electrooculograpy (EOG), etc. These sensors are often used for sleep studies in a clinical setting. However, while PSG systems are suitable for use in a clinical setting, such systems are typically impractical for use as a home screening tool.

Some alternatives to PSG require the patient to wear a specialized monitor such as a monitor with a flow sensor at home while sleeping. Such a sensor is adapted to receive respiratory flow information for detection of sleep disordered breathing events. The data is often sent to a third party, such as a physician, for further review. The cost and complexity of these monitoring alternatives can limit their widespread use. For example, requiring the patient to interact with a physician before obtaining a diagnosis can dramatically increase costs. Moreover, the equipment costs associated with the specialized monitoring device can also impede its widespread use. Thus, improvements may be desirable for providing a more cost-effective screener to assist in brining awareness of SDB to the general public.

Snore detection systems are available. For example, an application known as "SnoreLab" (available at http://www.snorelab.com) may be used to record the patient's bedroom sounds during a single night of sleep, extract the snoring sounds from other background sounds, and produce a graph of snore sound intensity from the snoring sound data. Another potential example is "SnoreClock" (available at http://www.ralphsapps.com/ index.jsp), which may also be used to record the patient's bedroom sounds during a single night of sleep, extract the snoring sounds from other background sounds, and produce graphs and statistics from the snore data.

These types of systems may be deficient in their ability to screen because they rely indiscriminately upon snoring data. This data set, by itself, is not likely to be sufficient for use within an accurate and dependable SDB screening tool. Moreover, because these applications are focused upon all snoring sounds, they exclude other forms of more reliable input signals.

Accordingly, improvements are desired for providing a cost effective screening tool to assist in raising awareness of SDB conditions so as to promote treatment of SDB among the general public. The realization of such a screener remains a considerable technical challenge.

EP 2457504 A1 relates to a sleep apnea syndrome testing apparatus including: an analyzing unit that analyzes every unit time a sound signal resulting from a subject during sleep; a determining unit that determines whether a unit time of the sound signal includes a breath sound or a characteristic sound produced when a patient with sleep apnea syndrome recovers from an apneic state into a breathing state, and the determining unit that determines that the sleep state of the subject in the unit time is any one of "breathing restored state," "state with breathing," and "state without breathing"; a storage unit in which a sleep state of the subject in each unit time is stored; and a detecting unit that detects an apneic state of the subject if a history of the sleep states of the subject indicates at least a transition from the "state without breathing" to the "breathing restored state".

WO 2012/155257 A1 relates to breathing disorder identification, characterization and diagnosis methods, devices and systems. It is said that methods, devices and systems may rely on the characterization of breath sound amplitudes, periodic breath sounds and/or aperiodic breath sounds to characterize a breathing disorder as obstructive (e.g. obstructive sleep apnea - OSA) or non-obstructive (e.g. central sleep apnea - CSA).

### 2 BRIEF SUMMARY OF THE TECHNOLOGY

The invention is set out in the appended set of claims.

The present technology concerns a user friendly, cost-effective SDB screening tool. Such a screening tool may assist in the detection of conditions, such as snoring, apnea, hypopnea, Cheyne-Stokes respiration and/or other SDB events, based upon an input signal from the user. Typically, the input signal may be an audio signal capturing sounds from the user.

In some versions, the screening tool may be implemented with an apparatus including a processor and a sensor, such as a microphone. In some such versions, the apparatus may be achieved with a programmed application, such as for a multipurpose processor that may operate in a mobile device, such as a smart phone or tablet having an integrated microphone or microphone accessory.

In some cases, a patient's snoring sounds are intermittently measurable throughout the night. Even if no snoring sounds are present, the patient's breathing can be laboured and loud for extended periods of time. Reliable respiration epochs (RREs) having certain prolonged periods of snoring or breathing can be isolated or determined such as if they characteristically have an input signal with a consistent respiration (snore or breathing) component. Because of this consistency, some input signals contained in an RRE are particularly useful for diagnosing certain conditions, like SDB events. The present technology is adapted to determine such RREs for event detection.

Accordingly, a first aspect of the present technology relates to apparatus, methods, and systems used to screen for targeted conditions, such as SDB.

Another aspect of present technology obtains an input signal associated with a patient sleeping and diagnoses a condition in a portion of the input signal.

Another aspect of the present technology utilizes input signals associated with a patient sleeping during one or more nights of sleep.

Another aspect of the present technology is a processor configured to receive an input signal, analyse the signal, and detect a condition based on a portion signal without the support of a physician or technician.

Another aspect of the present technology is a method for detecting an RRE within an input signal and determining if a condition is present in the RRE.

Another aspect of the present technology is a method for determining a number of SDB indicators based upon certain portions of the input signal, such as the RRE.

Some versions of the present technology include a method for detecting an event of sleep disordered breathing of a user. The method may include receiving, in a processor, an input audio signal representing sounds of the user during a period of sleep. The method may include determining, in the processor, from the received input audio signal, at least one reliable respiration epoch (RRE), the epoch including at least a period associated with audible breathing that may or may not include snoring. The method may include detecting, in the processor and based on data in at least one determined RRE, presence of a sleep disordered breathing event. The method may include outputting, from the processor, an indicator of the detected sleep disordered breathing event.

In some versions, each RRE may include a start phase and an event detection phase. The start phase may include a period of consistent audible breathing complying with at least one predetermined criteria such as to classify as a start phase. The event detection phase may include a period of consistent audible breathing that may include at least one of: a. one or more quiet periods of no audible breathing and of a length shorter than an apnoea event; b. one or more quiet periods of no audible breathing, the periods being long enough to be classified as an apnoea, but short enough to not be classified as a loss of audible signal event; and c. a quiet period of no audible breathing that is long enough to be classified as a loss of audible signal event. In this last case, the detection of such a period may be used to mark an end of the RRE. An event from the event detection phase may be classified as one of an apnoea event, hypopnea event and periodic breathing event. Since snoring is a type of audible breathing, any period of audible hearing may or may not include snoring.

In some versions, the method may include classifying an event as apnoea snoring by analysing at least one of audio frequency characteristics, audio level and timing characteristics of a snoring signal, and detecting apnoea snoring characteristics. The method may include classifying recovery breathing by analysing at least one of audio frequency characteristics, audio level and respiratory rate of a breathing signal and detecting recovery breathing characteristics. The method may include analysing the input audio signal to detect a recovery breath after a detected quiet period, to confirm an apnoea event. The method may include determining respiratory rate from the one or both of the audible breathing and the snoring. The method may include analysing the input audio signal to detect an elevated respiration rate after a detected quiet period to confirm an apnoea event. The method may include adjusting, with the processor, a gain control for the input audio signal so as to obtain a desired signal to noise for digital signal processing of the signal.

In some versions, method may include processing the audio signal over a fixed time interval to produce frequency components of the signal in a number of frequency bins for this time interval. The method may include analysing the frequency bins to remove any one or more frequency bins attributable to background sounds including at least one of: speech sounds, air handling equipment sounds, traffic sounds, and weather sounds. The method may include establishing, with the processor, a background noise level of the input audio signal and establishing, for remaining frequency bins, a noise floor threshold level for this background noise.

In some versions, the method may include determining a noise floor threshold by identifying, with the processor, one or more quiet frequency bins; setting the noise floor threshold as a function of amplitude of the signal in remaining quiet frequency bins; and wherein the method may further include utilising only these remaining quiet frequency bands to detect signals.

In some versions, the method may include determining at least one reliable respiration epoch by characterizing frames of the input audio signal based upon their correspondence with one or more signal types. The one or more signal types may include audible breathing, snoring, coughing, noise disturbance, speech, quiet and unknown. The method may include determining respiration rate for the determined at least one reliable respiration epoch.

Is some cases, the loss of audible signal event may be used to mark an end of the RRE, wherein the loss of audible signal event is a period of no audible breathing greater than a predetermined time. The method may include providing a notification of at least one of the following: one or more identified SDB events; measurement indices and statistics; historical data; snoring time; and other associated details.

In some versions, the start phase of the at least one RRE may comply with at least two or all of the following criteria: the RRE extends for at least a predetermined amount of time; the RRE includes at least a predetermined number of respiration cycles during the at least a predetermined amount of time; and the RRE includes at least a predetermined number of consecutive respiration events during the at least a predetermined amount of time. The predetermined amount of time may be two minutes. The predetermined number of respiration cycles may be six, over this predetermined amount of time of two minutes. The predetermined number of consecutive respiration events may be three during the predetermined amount of time of two minutes. In some cases, a loss of audible signal event is used to mark an end of the RRE, wherein the loss of audible signal event is a period of no audible breathing greater than a predetermined time.

Some versions of the present technology may include apparatus for detecting an event of sleep disordered breathing of a user. The apparatus may include a sound sensor configured to detect sound proximate to the sensor. The apparatus may include a processor coupled with the sound sensor. The processor may be configured to receive an input audio signal representing sounds of the user during a period of sleep from the sound sensor. The processor may be configured to determine from the received input audio signal, at least one reliable respiration epoch (RRE), the epoch including at least a period associated with one or both of audible breathing and snoring. The processor may be configured to detect, based on data in at least one determined RRE, presence of a sleep disordered breathing event. The processor may be configured to output an indicator of the detected sleep disordered breathing event.

In some versions, each/any RRE may include a start phase and an event detection phase. The start phase may include a period of consistent audible breathing complying with at least one predetermined criteria such as to classify as a start phase. The event detection phase may include a period of consistent audible breathing and that includes at least one of: a. one or more quiet periods of no audible breathing and of a length shorter than an apnoea event; b. one or more, quiet periods of no audible breathing, the periods being long enough to be classified as an apnoea, but short enough to not be classified as a loss of audible signal event; and c. a quiet period of no audible breathing that is long enough to be classified as a loss of audible signal event (e.g., the detection of such a period may be used to mark an end of the RRE).

In some versions, an event from the event detection phase is classified as one of an apnoea event, hypopnea event and periodic breathing event. The processor may be further configured to classify an event as apnoea snoring by analysing at least one of audio frequency characteristics, audio level and timing characteristics of a snoring signal, and detecting apnoea snoring characteristics. The processor may be further configured to classify recovery breathing by analysing at least one of audio frequency characteristics, audio level and respiratory rate of a breathing signal and detecting recovery breathing characteristics. The processor may be further configured to analyse the input audio signal to detect a recovery breath after a detected quiet period, to confirm an apnoea event. The processor may be further configured to determine respiratory rate from the one or both of the audible breathing and the snoring. The processor may be further configured to analyse the input audio signal to detect an elevated respiration rate after a detected quiet period to confirm an apnoea event.

In some versions, the processor may be further configured to adjust a gain control for the input audio signal so as to obtain a desired signal to noise for digital signal processing of the signal. The processor may be further configured to process the audio signal over a fixed time interval to produce frequency components of the signal in a number of frequency bins for this time interval. The processor may be further configured to analyse the frequency bins to remove any one or more frequency bins attributable to background sounds including at least one of: speech sounds, air handling equipment sounds, traffic sounds, and weather sounds. The processor may be further configured to establish a background noise level of the input audio signal and establish, for remaining frequency bins, a noise floor threshold level for this background noise. In some cases, to determine a noise floor threshold, the processor may be configured to: identify one or more quiet frequency bins; and set the noise floor threshold as a function of amplitude of the signal in remaining quiet frequency bins; and wherein the processor may be further configured to utilise only these remaining quiet frequency bands to detect signals.

In some versions, to determine at least one reliable respiration epoch, the processor may be configured to characterize frames of the input audio signal based upon their correspondence with one or more signal types. The one or more signal types include audible breathing, snoring, coughing, noise disturbance, speech, quiet and unknown. The processor may be further configured to determine respiration rate for the determined at least one reliable respiration epoch. In some cases, the start phase of the at least one RRE complies with at least two of the following criteria that may be evaluated by the processor: the RRE extends for at least a predetermined amount of time; the RRE includes at least a predetermined number of respiration cycles during the at least a predetermined amount of time; and the RRE includes at least a predetermined number of consecutive respiration events during the at least a predetermined amount of time. The predetermined amount of time may be two minutes. The predetermined number of respiration cycles may be six, over this predetermined amount of time of two minutes. The predetermined number of consecutive respiration events may be three during the predetermined amount of time of two minutes.

The processor may be configured to mark an end of the RRE upon detection a loss of audible signal event is used to, wherein the loss of audible signal event is a period of no audible breathing greater than a predetermined time. The processor may be further configured to generate an output notification (e.g., electronic communication, report and/or a display message) of at least one, or more, of the following: one or more identified SDB events; measurement indices and statistics; historical data; snoring time; and other associated details.

Some versions of the present technology may include a non-transient, computer-readable medium having processor control instructions retrievable therefrom that, when executed by a processing device, cause the processing device to perform a method for detecting an event of sleep disordered breathing comprising any one or more of the methods described herein.

The present technology may include a method for detecting an event of sleep disordered breathing. The method may involve receiving, in a processor, an input audio signal representing sounds of a user during a period of sleep. The method may involve determining, in the processor, at least one reliable respiration epoch from the input audio signal. The epoch may represent a period of laboured breathing and/or snoring from the period of sleep. The method may involve detecting, in the processor, presence of a sleep disordered breathing event in the at least one epoch. The method may involve outputting, from the processor, an indicator of the determined sleep disordered breathing event.

In some cases, the event may be any of an apnea event, hypopnea event and modulated breathing event. Modulated breathing may be understood to be a breathing signal with a modulated amplitude that is normally associated with Cheyne-Stokes Respiration. The event may be apnea snoring comprising a quiet period bounded by snoring. The method may involve determining respiratory rate from detected snoring. The method may involve determining a presence of snore.

In some cases, the method may involve analyzing the input audio signal to detect recovery breathing by analysis of audio level, rate and/or frequency after a detected quiet period. The method may involve analyzing the input audio signal to detect respiration rate after a detected quiet period to confirm an apnea event. The method may involve filtering the input audio signal to remove any one or more frequency components attributable to speech sounds, air handling equipment sounds, traffic sounds, weather sounds. The method may involve adjusting, with the processor, a gain control for the input audio signal. The method may involve establishing, with the processor, a noise floor and a noise floor threshold with the input audio signal. The determining a noise floor may include identifying, with the processor, one or more quiet frequency bands; and setting the noise floor threshold as a function of the identified quiet frequency bands. In some cases, determining at least one reliable respiration epoch may involve characterizing frames of the input audio signal based upon their correspondence with one or more respiration signal types. The one or more respiration signal types may include breathing, apnea snoring, non-apnea snoring, coughing, noise disturbance, quiet and unknown. The determining the at least one reliable respiration epoch may also involve determining, with the processor, a respiration rate from each epoch. In some cases, determining the at least one reliable respiration epoch may include determining, with the processor, that an epoch: commenced after a predetermined period of breathing and snoring; is part of a continuation of breathing and snoring that has continued for a prolonged period of time; and contains at least three consecutive respiration events every two minutes and an average of three respiration events per minute.

Some versions of the present technology may include apparatus for detecting an event of sleep disordered breathing. The apparatus may include a sound sensor configured to detect sound proximate to the sensor. The apparatus may include a processor coupled with the sound sensor. The processor may be configured to receive an input audio signal representing sounds of a user during a period of sleep, the input audio signal generated by the sound sensor. The processor may be configured to determine at least one reliable respiration epoch from the input audio signal, the epoch representing a period of laboured breathing and/or snoring from the period of sleep. The processor may be configured to detect presence of a sleep disordered breathing event in the at least one epoch. The processor may be configured to output an indicator of the determined sleep disordered breathing event.

In some cases, the event may be any of an apnea event, hypopnea event and modulated breathing event. The event may be apnea snoring comprising a quiet period bounded by snoring.

Optionally, the processor may be further configured to determine respiratory rate from detected snoring. The processor may be further configured to determine a presence of snore. The processor may be further configured to analyze the input audio signal to detect recovery breathing by analysis of audio level, rate and/or frequency after a detected quiet period. The processor may be further configured to analyze the input audio signal to detect respiration rate after a detected quiet period to confirm an apnea event. The processor may be further configured to filter the input audio signal to remove any one or more frequency components attributable to speech sounds, air handling equipment sounds, traffic sounds, weather sounds. The processor may be further configured to adjust a gain control for the input audio signal. The processor may be further configured to establish a noise floor and a noise floor threshold with the input audio signal.

In some versions, the processor may be configured to: identify one or more quiet frequency bands; and set the noise floor threshold as a function of the identified quiet frequency bands. In some cases, to determine at least one reliable respiration epoch, the processor may be configured to characterize frames of the input audio signal based upon their correspondence with one or more respiration signal types. The one or more respiration signal types may include breathing, apnea snoring, non-apnea snoring, coughing, noise disturbance, quiet and unknown. In some versions, to determine the at least one reliable respiration epoch, the processor may be configured to determine a respiration rate from each epoch. To determine the at least one reliable respiration epoch, the processor may be configured to determine that an epoch: commenced after a predetermined period of breathing and snoring; is part of a continuation of breathing and snoring that has continued for a prolonged period of time; and contains at least three consecutive respiration events every two minutes and an average of three respiration events per minute.

Some versions of the present technology may include a non-transient, computer-readable medium having processor control instructions retrievable therefrom that, when executed by a processing device, cause the processing device to perform any of the methods discussed herein for detecting an event of sleep disordered breathing.

Of course, portions of each aspect may form sub-aspects of the present technology. Also, various sub-aspects and/or aspects may be combined in various manners to constitute additional aspects or sub-aspects of the present technology.

Other features of the technology will be apparent from consideration of the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:
Fig. 1A illustrates an example processing device for receiving audio information from a sleeper that may be suitable for implementation of the processes of the present technology;
FIG. 1B is an example flowchart showing example processes suitable for some forms of the present technology.
FIG. 2A is a schematic illustration of a system in accordance with an example of the present technology.
FIG. 2B is conceptual diagram of a processing apparatus configured in accordance with some forms of the present technology.
FIG. 3 shows an audio signal of a typical breathing waveform of a person while sleeping. Upper section A shows breathing signal amplitude with time as received by a microphone and can serve as an input signal. Lower section B shows breathing signal frequency components from 0 Hertz to 4k Hertz with time of the above input signal.
FIG. 4 illustrates frequency component splitting of the signal of FIG. 3 after transform into discrete bands or frames. Upper section A shows breathing signal amplitude with time (an input signal). Lower section B shows breathing signal frequency components from 0 Hertz to 4k Hertz with time of the above input signal in frame periods (FF1..FF6) of a suitable time duration (e.g., about 250 ms each).
FIG. 5 illustrates a process for noise floor detection in the signal of FIG. 4. Upper section A shows breathing signal low signal amplitude with time (an input signal). Lower section B shows low frequency components from 0 Hertz to 4k Hertz with time of the above input signal.
FIG. 6A shows an audio signal and frequency spectrogram with snoring events during a period of snoring.
FIG. 6B shows an audio signal and frequency spectrogram with an example apnea event during snoring.
Fig. 6C shows the signal characteristics of an apnea snore associated with a person who may have apnea.
FIG. 6D shows an audio signal and frequency spectrogram with coughing events.
FIG. 6E illustrates frame filtering portions of an input audio signal. The frame periods FF1 to FF6 have a time duration (e.g., 250 ms) and the filter bands MB1 to MB4 for detection are in various ranges.
FIG. 6G shows an audio signal and frequency spectrogram with recovery breaths. In the example, a 30 second apnea followed by 5 recovery breaths are illustrated by the audio signal. The first breath is of a large amplitude and has many frequency components. The breathing rate is fast at 20 breaths per minute.
FIG. 6H shows an audio signal and frequency spectrogram during quiet breathing. Both the input signal amplitude and frequency components are low because the sound level is low.
FIG. 6I shows an audio signal and frequency spectrogram during repeated apneas. The signal graph illustrates apnea events of varying duration (30-50 seconds) each followed by several recovery breaths.
FIG. 7A illustrates respiration rate detection from frames of an audio power level signal. The signal power in each frequency band of interest may be computed and analyzed. The signal power may be computed on a frame by frame basis.
FIG. 7B shows the frequency components of the output signal after being filtered and analysed.
FIG. 8A shows audio signal with epoch characterization into reliable respiration epochs.
FIG. 8B illustrates interference detection from an audio input signal.
FIG. 9A illustrates event detection of apnea from reliable respiration epochs.
FIG. 9B illustrates event detection of Cheyne-stokes breathing from reliable respiration epochs.
FIG. 9C illustrates event detection of unusual modulated breathing or Cheyne-Stokes respiration.
FIG. 10 illustrates example output of detected sleep parameters over time from multiple sleep sessions with the system.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing particular examples discussed and is not intended to be limiting.

The following description is provided in relation to various forms of the present technology that may share common characteristics or features. It is to be understood that one or more features of any one exemplary form may be combinable with one or more features of another form. In addition, any single feature or combination of features in any of form described herein may constitute a further exemplary form.

### 4.1 SCREENING AND DIAGNOSIS

The present technology concerns a screening tool that may be implemented to assist in preliminary identification of a condition, such as a Sleep Disordered Breathing (SDB) condition. A patient with acute obstructive sleep apnea, for example, tends to emit loud and persistent snoring sounds or other laboured breathing for a large percentage (>50%) of each night. A screening tool of the present technology may include processes to determine the presence of events indicative of such conditions in a portion of an input sensor signal, such as by determining one or more reliable respiration epochs (RRE) from an audio signal of a microphone.

The basis of using RRE periods is in that snoring and other audible breathing (i.e., any breathing that can be heard, such as by a microphone, whether normal, laboured or otherwise) tends to be present in the breathing of people with SDB or CSR-related (central) apnea or hypopnea. Because of the specific morphology of the respiratory signal associated with central apneas or hypopneas, they are also referred to as "periodic breathing" events or "modulated breathing" events. Thus, for a more reliable detection of apnea events, the processing analyzes for quiet periods (alleged apneas), which may be partially or completely surrounded (i.e., before and/or after) by periods of clear audible breathing or snoring. Thus, the RRE can represent one or more periods of clearly detected audible breathing surrounding, or being adjacent to, a period of silence that is long enough to be an apnoeic event. If a period of silence is a part of such an RRE, or a number of periods of silence are interspersed with recovery breathing and snoring, there is a high likelihood that an apnoeic event exists/has occurred.

If a detected silent period is not part of an RRE (it is not surrounded by, or adjacent to, a period or periods of audible breathing), then the silence is more likely to represent normal inaudible breathing.

The presence of an RRE can be detected in input signal based upon its characteristics. In some forms of the present technology, an RRE can be detected based upon a respiration rate in the input signal and a portion of the input signal that corresponds with respiration, such as breathing, snoring, or quiet. In most cases, each RRE starts with a period of consistent audible breathing, including snoring, and may end on the last repetitive and uninterrupted breathing or snoring signal before a prolonged period of silence, noise, infrequent breathing or infrequent snoring. In the middle section , the event detection phase, event detection can include detection of one of the following: a/ A period of consistent or inconsistent audible breathing that may or may not include snoring, but with no apnoea; b/ A period of consistent or inconsistent audible breathing including snoring that might or might not contain apnoea events; and c/ A period of No breathing / silence which is both long enough to be considered an apnoea (e.g., in a range of about 10 to 120 seconds), but short enough NOT to be considered as a "LOSS OF AUDIBLE SIGNAL" (e.g., time periods well above about 120 seconds). If the signal is too long and can be considered as a loss of audible signal, the RRE may be marked as ended.

Some forms of the present technology rely exclusively upon the RRE for determination of a condition, while other forms also rely upon various SDB indicators. These supplemental indicators may be based upon any portion of the input signal, including any RRE or non-RRE portions.

In other words, an RRE can be considered a reliable respiration epoch determined from the input audio signal. It might be anything from a period of perfectly normal audible breathing that lasts for just 10 minutes during a whole night of inaudible breathing, to a brief period of consistent snoring followed by a whole night of apnoeic events.

Important considerations include that:
1. There is/are sufficient source(s) of audible signals (inconsistent / consistent) (normal/ laboured-audible) (breathing /snoring) to maintain the breath detection;
2. That the apnea events immersed in this can be reliably detected; and/or
3. inaudible breathing is the enemy of the detector. It provides no capability to detect apnea.

In some cases, the RRE may be determined according to one or more of the following criteria:
(a) A **START** section or phase comprising a period of consistent audible breathing, which may include snoring.
(b) An **END** section or phase comprising also a period of consistent audible breathing, which may include snoring.
(c) A **MIDDLE** section or phase comprising one or more of the following:
   1. A period of consistent audible breathing including snoring with no apnoeas.
   2. A period of consistent audible breathing including snoring that might contain apnoea events;
   3. A period of No breathing / silence which is:
      i) long enough to be considered an apnoea, but
      ii) short enough NOT to be considered "LOSS OF AUDIBLE SIGNAL".
      A period that is long enough to be considered "LOSS OF AUDIBLE SIGNAL" may be used to terminate the RRE and classify it as invalid and unusable as a potential apnoea detection period.

Typically, detection of such a start section and middle section can result in an indication/occurrence of an RRE. Further detection of an end section can indicate ending of an RRE.

### 4.2 SCREENING, MONITORING, DIAGNOSIS SYSTEMS

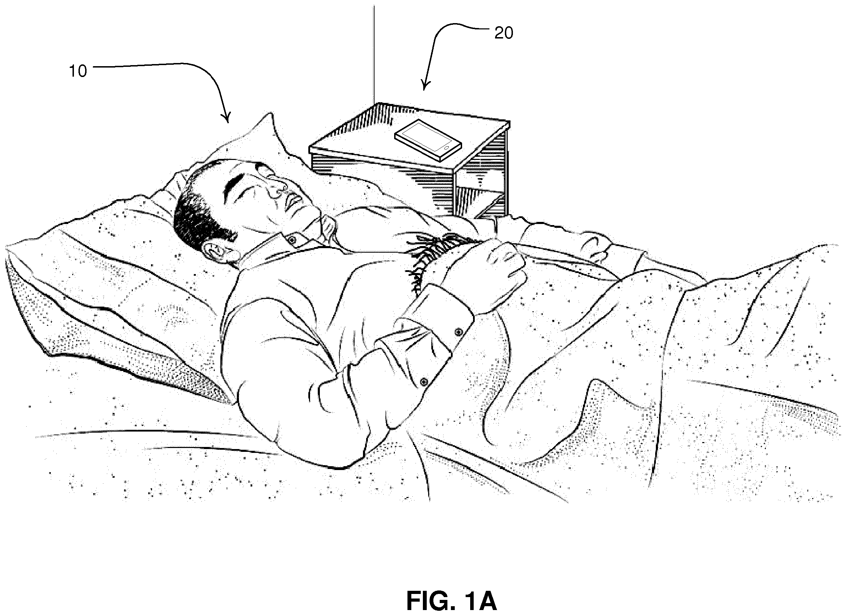

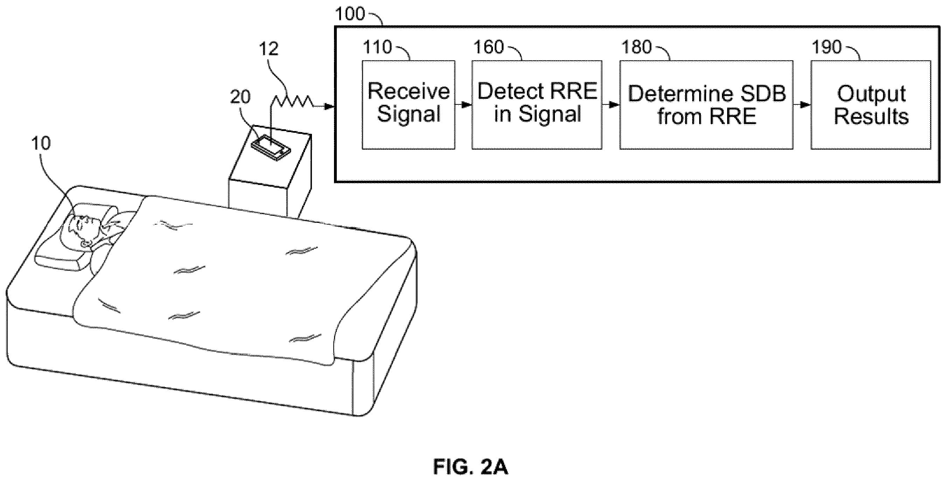

An example system according to the present invention is illustrated in FIG. 1A and FIG. 2A. As shown, a user 10 is near a screening device 20, such as a mobile phone or tablet, having a microphone and one or more processors. The processor(s) is/are configured to receive an input signal 12, such as a sound or audio level signal from a microphone of the screening device, execute a screening process 100, and output indicia to aid in detection of various events associated with SDB.

### 4.2.1 Screening Device 20

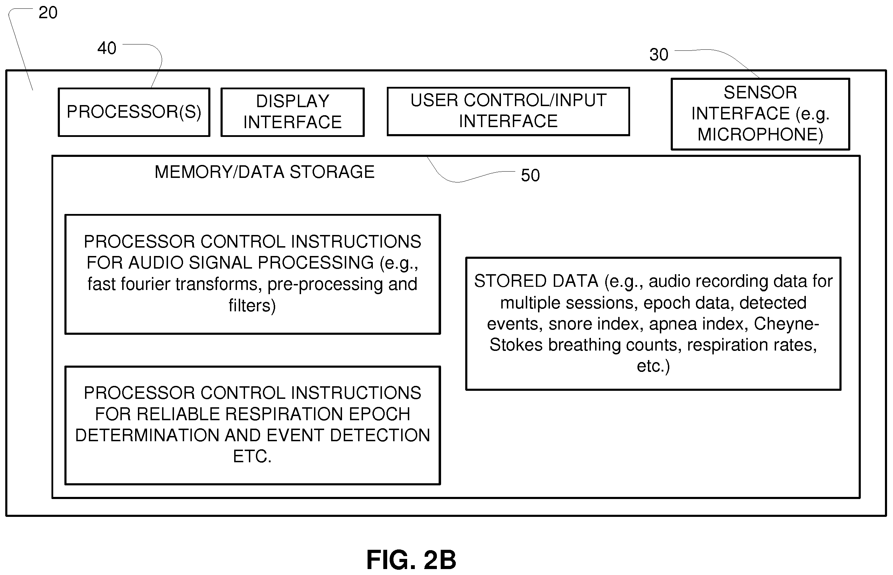

Device 20 may be adapted to provide a cost-effective and user friendly option for evaluating potential for SDB in input signal 12, shown in FIG. 2. In some forms of the present technology, signal 12 is an audio level signal such as from an audio recording that may be generated by a microphone. Thus, the screening device 20 may be a smartphone or tablet, for example. The screening process 100 may be affected by a processor control application or program, i.e. one that is run within the screening device 20. However, these processes may be implemented by other processing devices. For example, process 100 may be executed by elements of one or more medical devices, such as an RPT device, a general purpose computer or a specific purpose computer etc.

Thus, the processing device or apparatus may include integrated chips, a memory and/or other control instruction, data or information storage medium. For example, programmed instructions encompassing the assessment/signal processing methodologies described herein may be coded on integrated chips in the memory of the device or apparatus to form an application specific integrated chip (ASIC). Such instructions may also or alternatively be loaded as software or firmware using an appropriate data storage medium. Optionally, such processing instructions may be downloaded such as from a server over a network (e.g. an internet) to the processing device such that when the instructions are executed, the processing device serves as a screening device.

The screening/processing device 20 may include a number of components as illustrated by FIG. 2B. The device 20 may typically include a microphone or sound sensor 30, a processor 40, and a memory/data storage 50. Other components may also be included, such as a display or display interface and user control/input interface (e.g., touch screen or keyboard, etc.)

Any of these sub-elements of device 20 may be integral with or operably coupled with device 20. For example, microphone or sound sensor 30 may be integral with device 20 or coupled with device 20 such as through a wired or wireless link (e.g., blue tooth etc.).

### 4.2.2 Screening Process 100

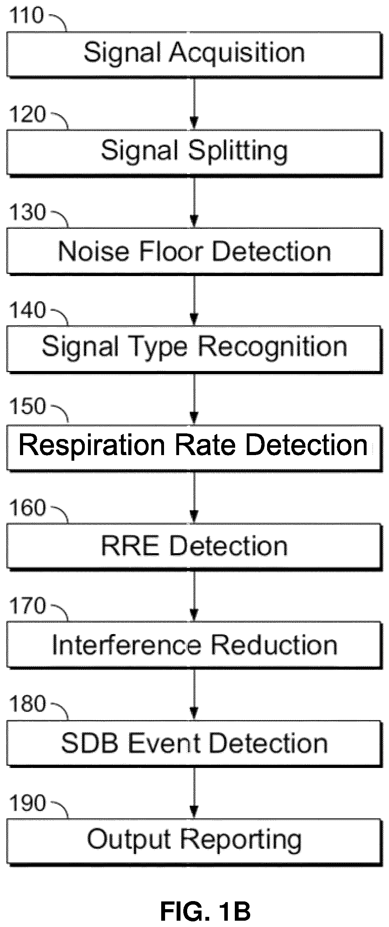

Examples of the present technology utilize one or more algorithms/processes within a screening process 100 to detect RREs within an input audio signal and determine SDB events from the RRE(s) over one or more sleep sessions with the screening device. Example processes may be considered in reference to FIGs. 1B and 2A. Such processes can be run on the screening device 20 and/or on external processing devices.

For example, screening process 100 may be characterized by several sub-processes. As shown in Fig. 2B, the screening device receives one or more input signals for RRE determination such as from one or more sleep sessions at 110-150. The device may then determine one or more RREs in the input signal(s) at 160-170. The device may then detect SDB related events in the RREs at 180. The device may the generate output indicative of the detected events at 190, such as for showing results of the user 10 of FIG. 1 or FIG. 2A.

### 4.2.2.1 Receiving an Input Signal (110-150)

### 4.2.2.1.1 Signal Acquisition 110

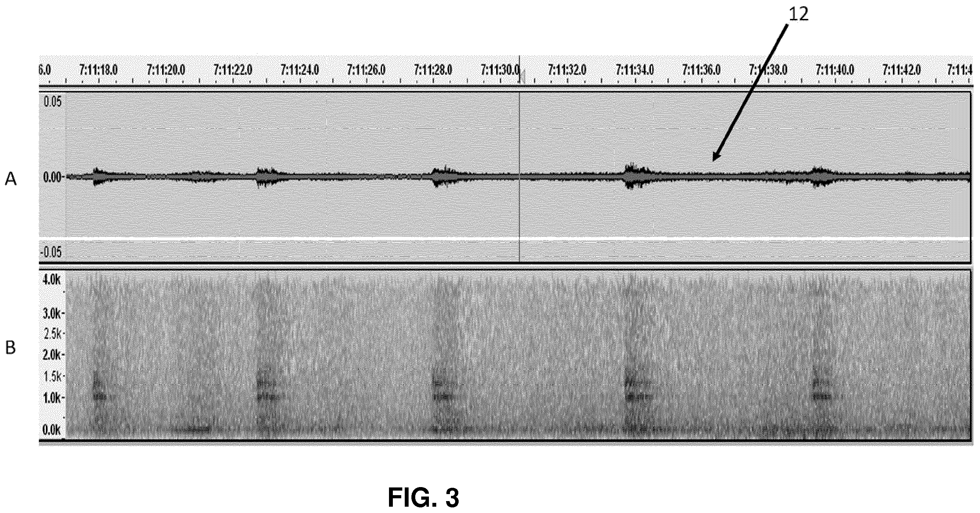

As illustrated in the flow chart of Fig. 1B, an input audio signal is obtained in signal acquisition process 110 of screening process 100. The signal, which may be recorded data sampled from a microphone or a microphone audio signal, represents sounds of a user 10 in association with a period of sleep T (FIG. 3). In FIG. 2A, input signal 12 is received at device 20 by a sound sensor 30 or microphone that is operably coupled with the device 20.

Input signal 12 may be an audio signal at least representing sounds of the user's breathing and snoring signals as well as other recorded sounds of a sleep session (e.g., environmental noise). An exemplary signal 12 is illustrated in FIG. 3 as the typical laboured breathing should form of user 10 sleeping during period T. Period of sleep T may comprise at least a portion of one night of sleep. Since RRE may represent only a small part of any sleep period T, as described below, input signal 12 may be received from a plurality of sleep periods T (e.g., sleep sessions). Accordingly, in some cases, further analysis may proceed after verification that sufficient amount of audio breathing data exists for performing subsequent processing, such as detecting RRE.

Generally, sound sensor responds to sounds in the vicinity of the sensor and generates a sound signal which may be sampled and recorded by the screening device 20. Optionally, signal acquisition at 110 may include automated gain control for increasing the quality of input signal 12. For example, processor 40 of device 20 may be configured to adjust the gain of the sensed audio level of signal 12 from microphone. For example, if clipping occurs or the noise floor (discussed in more detail herein) is too high, then the gain may be decreased. Conversely, if the audio level is too low, then the gain may be increased to improve the quality of signal 12 by increasing the audio level until a noise floor threshold is reached.

### 4.2.2.1.2 Signal Splitting 120

A signal spitting process 120 may then be performed to further improve the quality of input signal 12. Desirably, this process may be implemented to allow any persistent and unwanted audio noise components to be removed from signal 12 prior to RRE detection.

In some versions of the present technology, process 120 involves splitting the input audio signal into frames. For example, the input signal 12 may be sampled at 8 kHz or greater with a resolution of 8 bit linear PCM or greater, for example. The audio signal may be stored on the memory storage 50 of device 20 as a set of duration frame files (or frames) for analysis on a frame file by frame file basis. Each duration frame file may contain about 0.1 to 0.25 seconds of the input signal 12 or other suitable time period. The files may be stored on memory storage 50 together with any of the markers described below.

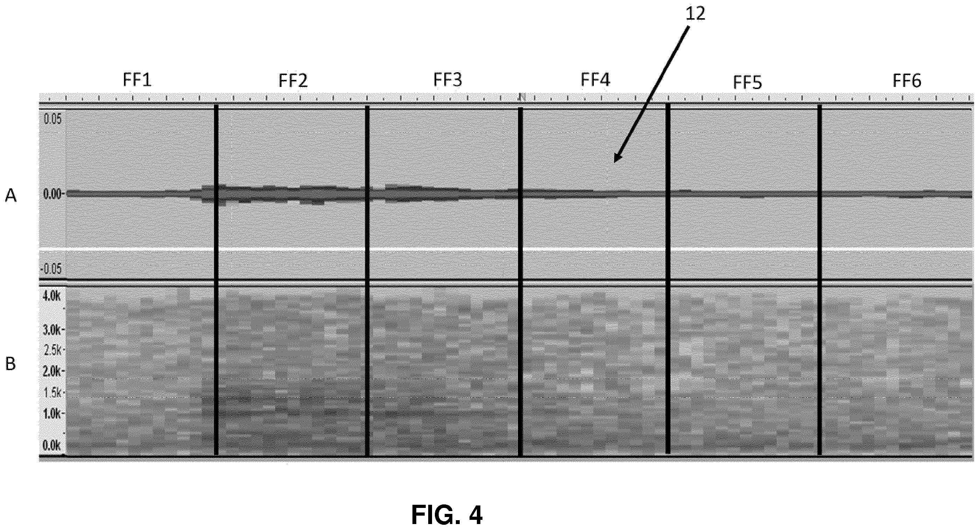

As illustrated in Fig. 4, each frame file, through transform such as Fast Fourier transform, is split into its frequency components. The example of Fig. 4 shows six 0.25s frames, each shown in one of rectangular box FF₁ FF₂, FF₃, FF₄, FF₅, FF₆ that includes the frequency components of the respective frame. For example, the resulting FFT may split the components typically into 1024 frequency components, each of a band width of 4 Hz. These are normally referred to as bins or frequency bins. Some of these frequency components may be used to diagnose SDB, whereas others may not. Thus, frequency components / bins associated with frequencies other than breathing and snoring may be removed.

### 4.2.2.1.3 Noise Floor Establishment 130

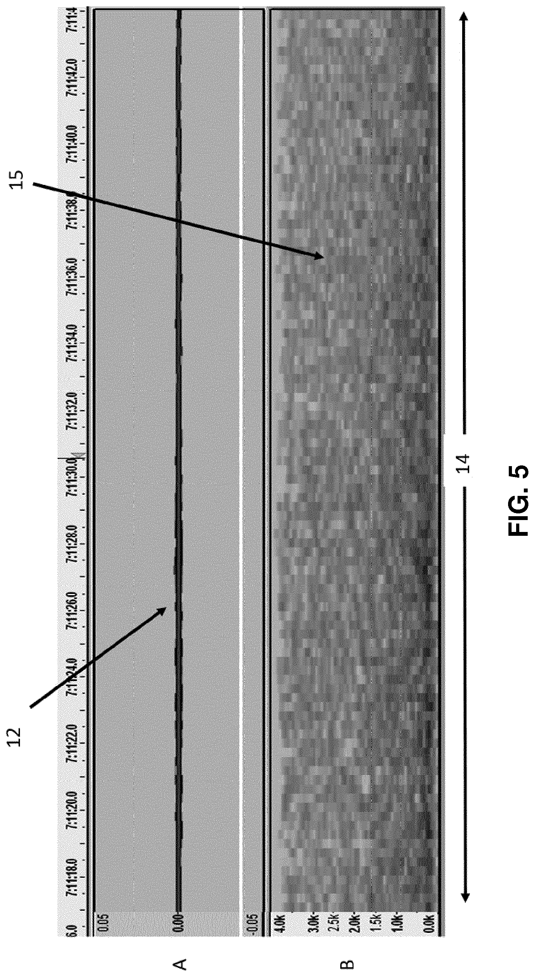

Optionally, a noise floor establishment process 130 may be executed to establish a background noise floor and noise floor threshold for each frame. Such a process may improve the quality of signal 12 so as to account for variations in microphone type and/or room background noise conditions. The noise floor threshold may be established with reference to a quiet interval 14 (FIG. 5), which is a period of the input signal 12 with a prolonged absence of audio noise. The audio level associated with the quiet interval may then be used to set the noise floor threshold.

For example, process 130 initially may involve integrating the magnitude of the frequency components of a predetermined number of frames to identify a number of quiet frequency bands. As illustrated in FIG. 5, the frequency components of quiet interval 14 have been integrated to identify a quiet frequency band 15. In Fig. 5, top part A illustrates the audio signal amplitude against time during a quiet period. In the bottom part B, the frequency component intensity over time is illustrated. The frequency component intensity is represented by the intensity of the colour used - light meaning no components in this area of frequency and time, whilst dark meaning components present at this frequency and time.

Frequency bands with persistent high noise (high noise in a common band of multiple frames) can be disregarded. For example, frequency components that are less than 80 Hz can be a major contributor to noise floor and 1/f noise. Examples include background sounds like air conditioning, fans and other mains powered equipment that have low frequency acoustic noise components; the ambient audio noise level has a strong low frequency component due to low pass filtering. Low-frequency noise is common as background noise in urban environments, and as an emission from many artificial sources such as road vehicles, aircraft, industrial machinery, and air movement machinery including wind turbines, compressors, and ventilation or air-conditioning units. The effects of low-frequency noise are further exacerbated due to its efficient air propagation, and the reduced efficacy of the walls of many structures/dwellings in attenuating low-frequency noise compared with higher frequency noise. In addition, the measurement system amplifiers used to detect the acoustic noise and to generate a detectable and useful electrical signal can have a 1/f noise characteristic (pink) noise. Thus, process 130 may disregard bands less than 80 Hz.

However, frequency components that are less than 80 Hz may be associated with certain types of snoring, such as apnea snoring. Continuous noise floor components such as mains hum are filtered out, variable noise components such as snoring are retained.

### 4.2.2.1.4 Signal Type/Sound Recognition 140

Signal type process 140 may be applied to characterize portions of input signal 12 that correspond with one or more exemplary signal types for further processing. For example, process 140 may characterize frames of the input signal 12 based upon their detected characteristics.

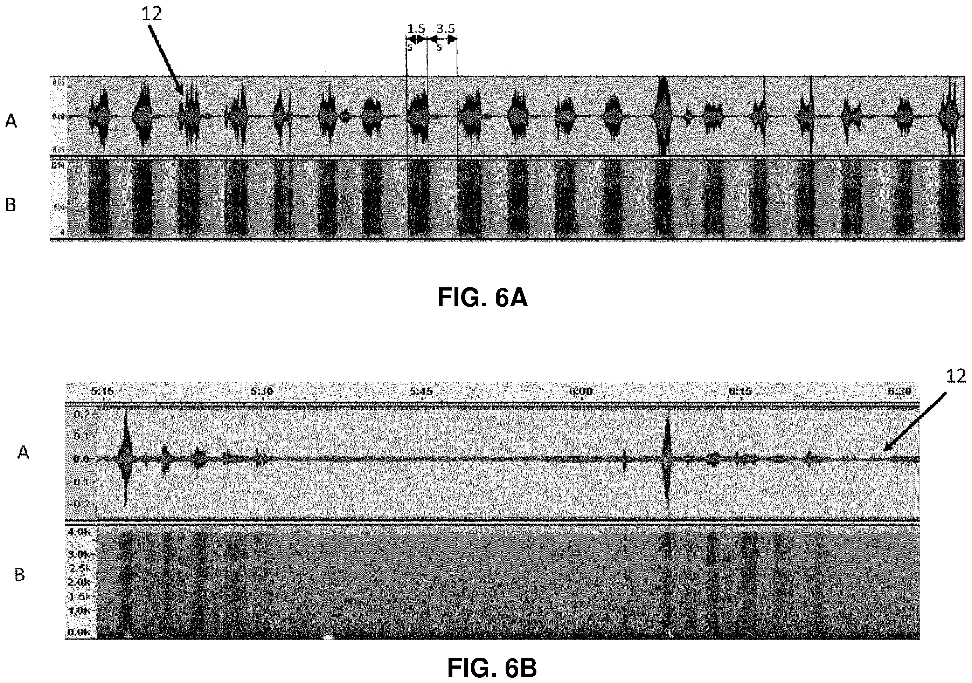

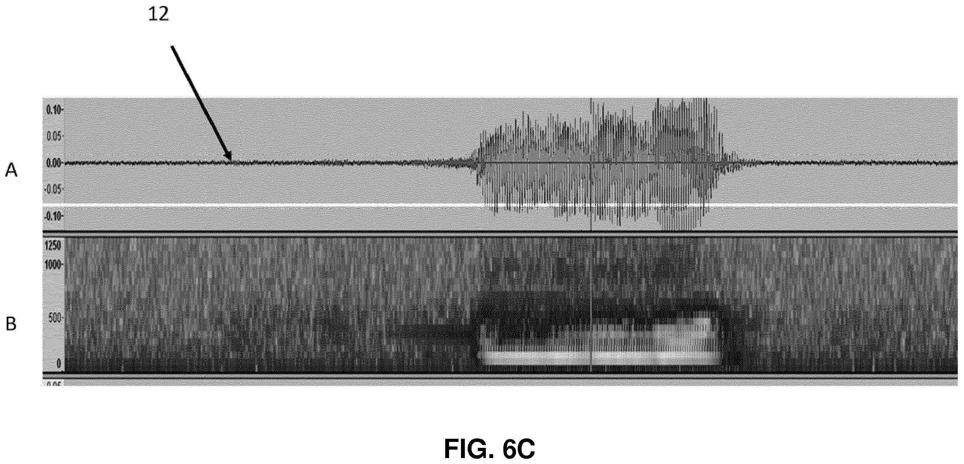

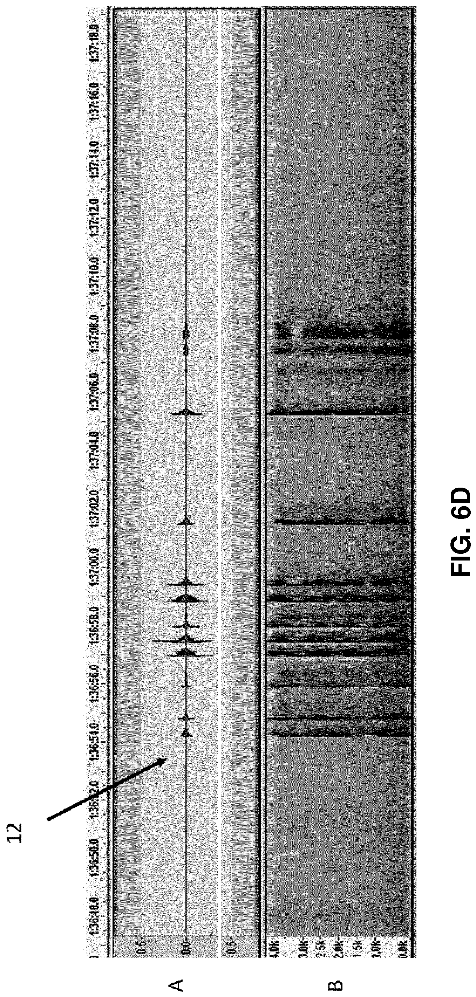

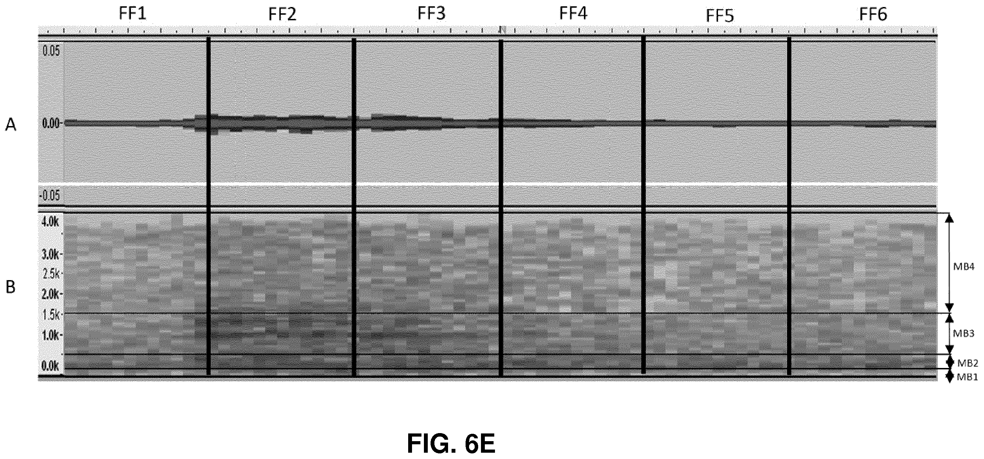

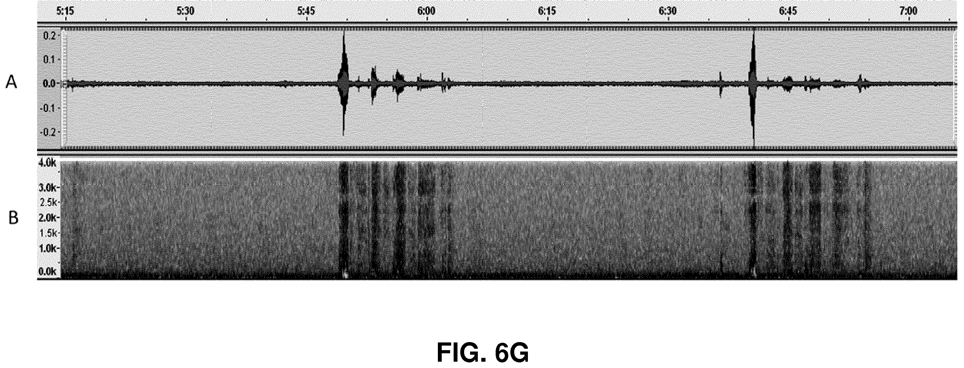

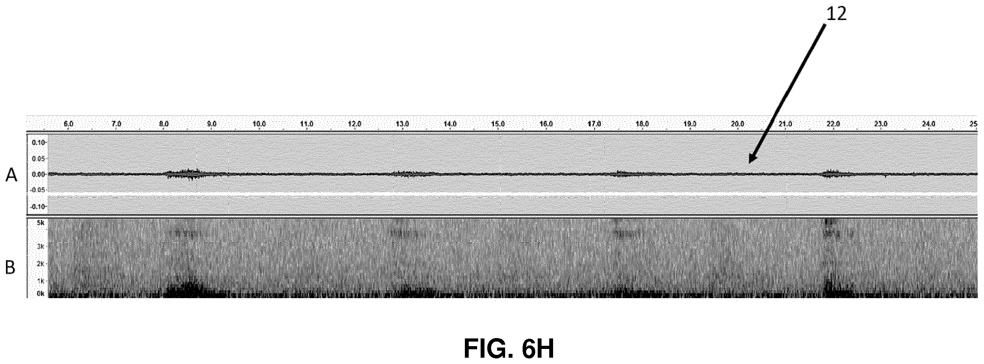

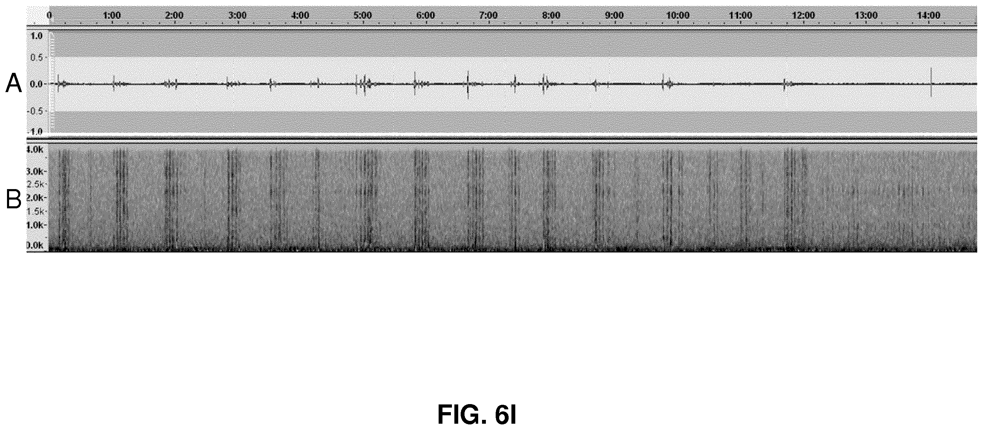

As illustrated in FIGs. 6 A-D portions of signal 12 associated with each of quiet breathing (FIG. 6H), snoring (FIG. 6A, 6C), apnea snoring (FIG. 6B), and coughing (FIGs. 6D) may be distinguishable from each other in terms of frequency, time and amplitude.

The snoring signal of FIG. 6A, for example, is associated with a respiration rate of about 12 breaths per minute, and comprises an alternating pattern of about 1.5s sound, high signal amplitude, and 3.5s quiet, low signal amplitude, and frequency components of snoring (low frequency, 200-500Hz). Snoring can have a number of frequency components with a few different sources: throat, mouth, nose and can depend on the person and type of snore. Specifically the very high components around 2 to 4kHz tend to result from a whistle / hiss type sound generated by nasal or mouth breathing and its harmonics Snoring may be loud (i.e., a high input signal amplitude that is associated with audio level).

Whereas the acoustically quiet breathing of Fig. 6H may have high frequency, 500Hz-4kHz) and quiet (low input signal amplitude) with about 1.5 seconds of sound and about 3.5 seconds of quiet. In this regard, Fig. 6H is an input signal with about a 12 breath per minute rate where there is approximately 1/3 snore and 2/3 quiet.

To provide further examples, the apnea snoring signal of FIG. 6C may be visible during snoring signals or laboured breathing. It differs from a normal snore in that it has a higher content of low frequency components, and tends to be of a higher signal level (louder). An apnoea snore is an indication that the snoring person may be prone to sleep apnoea. Thus, while in some cases as discussed herein, apnea snoring may be detected with any type of snoring detection but in its particular relation to, or bounding of, a quiet/apnoeic period, in some cases apnea snoring may be detected merely from the particular nature of the apnea snoring sound characteristic(s) as it differs from normal snore. In still some further cases, both analyses may be applied to detect apnea snoring event(s).

In comparison, the coughing signal of FIG. 6D is associated with a random pattern of about 0.5s of sounds and 0.5s or greater of quiet and frequency components of 100-4kHz with more high frequency energy than snoring.

As illustrated in Fig. 6G, showing the input signal amplitude verses time (minutes/second) in part A and frequency components in part B, a recovery breath which is typically associated with the end of an apnea may be a gasp with snoring rather than just snoring. These recovery breaths may be characterized by a high audio level and high frequency (e.g., the above statements are relative to the average snore or breathing acoustic level and frequency. The amplitude of a snore is normally reasonably consistent within an epoch, recovery breaths normally start with a period of apnea followed by a higher than average first breath/snore [e.g., twice the average amplitude]. It also may contain more harmonic components and so may have more high frequency components, e.g., average fundamental at 300-500Hz with harmonic components at 800Hz, 1200Hz etc.) and by faster than normal rate (e.g., again compared with average breathing rate of say 12bpm; the recovery breath could be at 20 to 30bpm). The recovery breath / recovery snore has an acoustic fingerprint that can be detected by knowing the user's average breathing or snoring audio characteristic during the overall nights testing.

An exemplary listing of signal types and their characteristics are tabulated below. Other noises are also listed.

| **Signal Type** | **Frequency Band (Hz)** | **Duration (s)** | **Period (s)** | **Relative Amplitude** |
|---|---|---|---|---|
| Normal Breathing | 500 - 4000 | 1s | 5s | Low |
| Recovery Breath | 100 - 4000 | 1s | 3s | High |
| Snoring | 100 - 500 | 1s | 5s | Medium |
| Apnea Snoring | 20 - 160 | 1.5s | 5s | High |
| Coughing | 0 - 4000 | 0.5s | >1s | High |
| Quiet interval | 0 - 4000 | 0s | Continuous | Noise floor |
| Speech Noise | 100 - 4000 | Random | Random | Medium |
| AC noise | 0 - 100 | Continuous | Continuous | Medium |
| Traffic noise | 0 - 500 | 5s - 10s | 10s | Low |
| Unknown | 0 - 4000 | Random | Random | Variable |

To distinguish between these exemplary signal types, process 140 may include frame filtering and frame analysis.

For example, the frame filtering may be implemented to group the frequency components of the input signal into categories of audio frequency bands based upon signal energy in the bands. The audio signal level may then be summated for each such band. For example, the frames of the input signal may be characterized into the following bands:
(MB1) the main apnea snoring signals energy (e.g., 40 frequency components (4hz each) from 0Hz to 160Hz);
(MB2) the main non-apnea snoring signals energy (e.g., 100 frequency components (4hz each) from 100Hz to 500Hz);
(MB3) the low frequency breathing energy (e.g., 250 frequency components (4hz each) from 500Hz to 1500Hz); and
(MB4) the high frequency breathing energy (e.g., 625 frequency components (4hz each) from 1500Hz to 4000Hz).
Each of these main bands (marked as MB1-MB4) is illustrated in FIG. 6E with reference to the six 0.25s frame files (FF1-FF6) introduced in FIG. 4.

In the frame analysis, a select number of frames in one or more of the main audio frequency bands described above are analysed for correspondence with one or more of the exemplary signal types noted above. A typical example of a period of apnea followed by a series of recovery breaths which are accompanied by loud snoring is depicted in FIG. 6G. This can be determined in an automated system by applying the rules of the exemplar signal table above to the actual signal characteristics found during these selected frames. The analysis can provide a probability for each signal type based on the table. The state of the signal can be chosen based on the highest probability signal type from the analysis. The accuracy of the automated assessment can be confirmed by listening to the audio signals. Such feedback can be really helpful in fine-tuning the parameters of the detecting system.

As with the previous examples, the processing includes analysing groups of frames (each having a duration of, for example, 0.25s), over a sliding window (e.g., 5s interval) to determine whether its characteristics (e.g., frequency, duration, period, and/or audio level) correspond with the characteristics of one or more of the signal types noted in the previous table. If a correspondence is found, then that group may be marked by the designated type. For example, processor 40 may be configured to store any frames corresponding with signal types of any one of breathing, snoring, apnea snoring, coughing, quiet interval, noise disturbance (e.g., speech noise, air handler/conditioning AC noise, traffic noise) and unknown.

### 4.2.2.1.5 Respiration Rate Detection

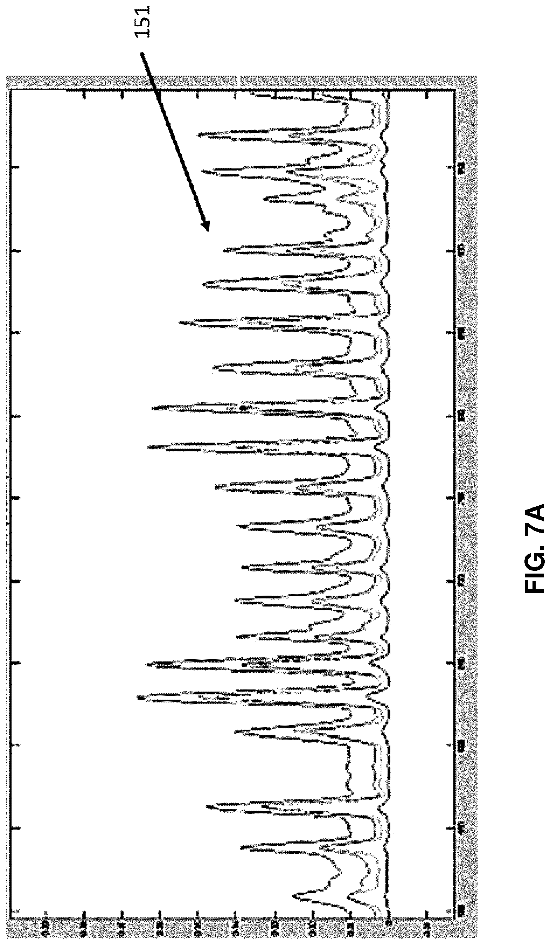

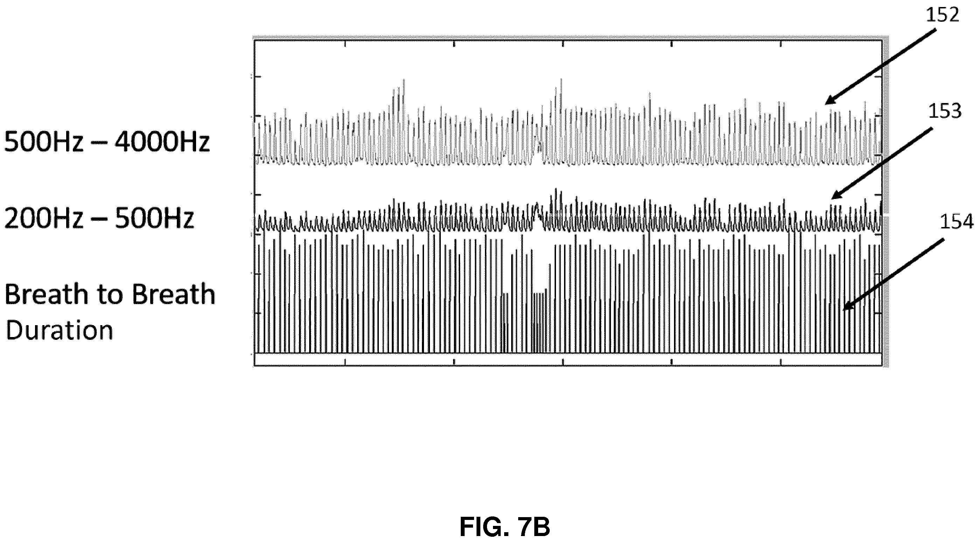

Optionally, the process 100 may include a respiration rate detection process 150. Consistent epochs of breathing and snoring enable the system to determine the respiration rate. For example, with frames characterized by breathing or snoring, a respiration rate may be determined. As illustrated in FIG. 7A, a signal 151 may be generated from the audio power level in the characterized frames for each of the frequency bands. Once generated, the frequency components of the generated signal are filtered and analysed using FFT to determine the presence of respiration frequency components (e.g., respiration rates in respiration frequencies such as breathing or snoring). An illustration of this frequency analysis is shown in FIG. 7B, which illustrates a breathing signal 152, a snoring signal 153, and a respiration rate 154.

Optionally, detection of a respiration rate may assist with verification that a frame is part of a RRE, as will be described later in the text.

### 4.2.2.2 Detecting an RRE in the Input Signal (Steps 160-170)

As described above, an RRE is a prolonged epoch of snoring or breathing signals having significant characteristics. Several indicative characteristics of an RRE may be:
(1) commencement of audible breathing or snoring signals after a number of seconds, typically 60 seconds, of repetitive and uninterrupted breathing or snoring signals;
(2) continuation of said signals for a prolonged period of time, such as 10 minutes or greater; and/or
(3) maintenance of (i) at least three consecutive respiration events every two minutes, and (ii) an average of three respiration events per minute.

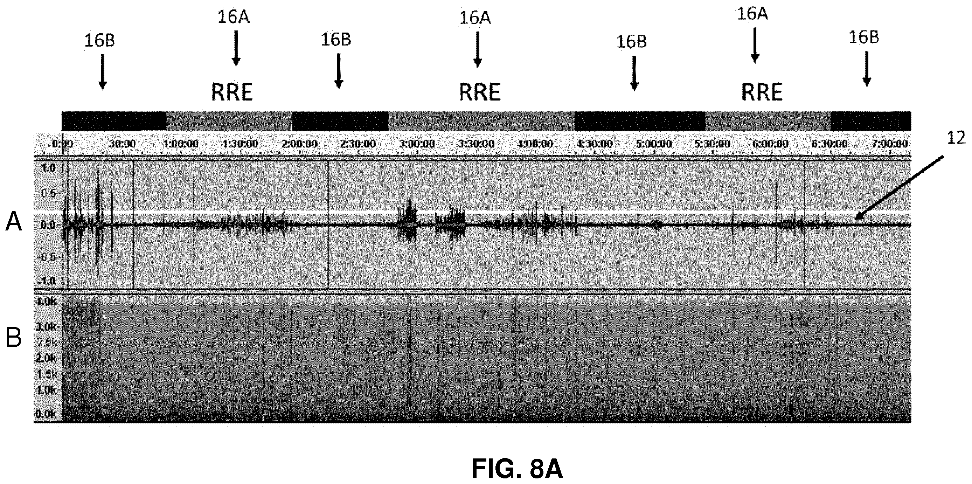

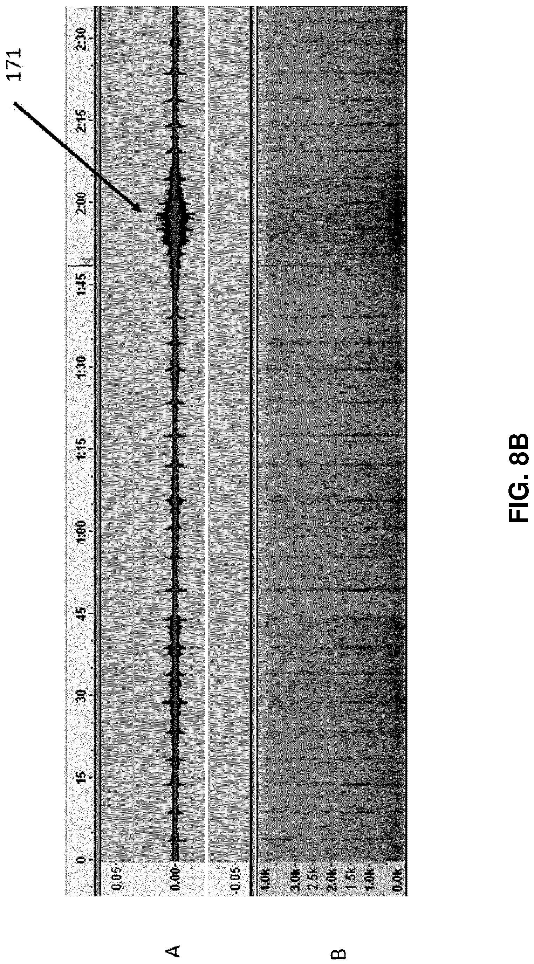

FIG. 8A illustrates determination of an RRE in a portion 16 of input signal 12 that has each of these three RRE characteristics. Portion 16 of signal 12 is shown as itself having two portions 16A, 16B. Each portion 16A represents detected breathing or snoring signals, that meet the requirements of an RRE while each portion 16B represents a detected quiet interval. For example, these portions 16A may satisfy the RRE start, middle and end criteria. Each of these two portions 16A-B may be detected in the determination of an RRE.

### 4.2.2.2.1 RRE Detection 160

Generally, characterization of a frame as part of an RRE may be based on detection of a respiration rate (such as a rate in a range attributable to breathing and/or snoring) in the particular frame. In some versions of the present technology, a RRE detection process 160 may involve analysing each potential frame on a frame-by-frame basis of an input signal to determine if the signal levels of a current frame commenced after a predetermined number of seconds of repetitive and uninterrupted "breathing" or "snoring" signals, such as those depicted in FIG. 7B. The analysis refers back to the signal characteristic table and is based on the signal level in the two frequency bands as well as their cadence (presence absence ratio) and periodicity, which define the signal characteristics (snoring, breathing etc. The expression "uninterrupted" here means to refer to breathing without long gaps that are considered an apnoea or a loss of signal.

This may be determined from an evaluation of signal levels of a preceding frame or frames. If the predetermined number of seconds is on the order of about 60 seconds or longer, then an RRE in the current frame is likely and it may be marked as an RRE. In some versions of the present technology, this condition may be sufficient for detection of an RRE. In other forms, further conditions may be implemented.

For example, an RRE may be determined by analysing each potential frame of an input signal to determine if its signal levels represent a continuation of breathing, laboured breathing or snoring for period of time. For example, an RRE may be determined for a current frame if the breathing or snoring exists for a prolonged period of time, such as on the order of minutes (e.g., 10 minutes) or greater as determined from an analysis of subsequent frames and/or previous frames. As a further example, if the respiration frequency components of a sequence of frames have been maintained (i) for at least three consecutive respiration events every two minutes, at (ii) an average of three respiration events per minute. In this example, the duration and consistency of the respiration rates are utilized to further confirm the presence of an RRE with the sequence of frames.

A frame may be marked as an end of an RRE if the respiration components contained therein (FIG. 7B) include the last repetitive breathing or snoring signal before a prolonged period of silence, noise, infrequent breathing or infrequent snoring, such as by consideration of a subsequent frame. Here "silence" or a "silent period" is intended to mean a period when the audio signal is below a signal threshold and there are no audible signals that meet the SNORE or AUDIBLE BREATHING detection requirements provided in the table.

Each frame that meets the three characteristics may be marked as RRE frames in memory storage 50 of device 20. Conversely, if one of the characteristics is not met, then the frame may be ignored or marked as non-RRE.

### 4.2.2.2.2 Noise Interference Reduction 170

Unwanted noise can hinder the diagnostic capabilities of an RRE. Therefore, to enhance the signal quality, a noise interference reduction process 170 may be performed on one or more of the frames marked as an RRE. Process 170 is illustrated in FIG. 8B, which illustrates undesired noise 171 within a portion 18 of input signal 12 of an RRE. Process 170 removes at least a portion of any unwanted noise interference from portion 18. For instance, low frequency noise, such as traffic or high frequency noise such as wind, can be at least partially removed (i.e. filtered out) from the FFT of the signal, whilst continuous tones may be completely removed.

Process 170 may remove different types of noise. For example, process 170 may remove continuous noise, such as that produced by air conditioning equipment or calm wind. For example, process 170 may detect a noise frequency during a quiet breathing interval. Thereafter, the frequency of detected noise may be filtering out of the RRE by FFT.

By way of further example, process 170 may remove periodic noise, such as traffic noise. For example, periodic noise may be detected by detecting an increase in the noise floor (such as by comparison with a previously determined noise floor threshold). The frequency of the periodic noise may then be detected and removed from the frames of the RRE by filtering such as with FFT filtering. In some versions, the frames of the RRE containing the detected periodic noise or detected noise of a short time period may be marked for exclusion from the RRE or masked as a noisy portion of the RRE (e.g., periodic noise of less than about 2 to 10 seconds).

By way of further example, process 170 may remove random noise, such as that created by speech or gusty wind. If the duration of the detected random noise is short (e.g., about 1 to 10 seconds), then the noise may be removed from the RRE by filtering the frames of the RRE. Alternatively, the frames of the RRE may be marked as containing noise and removed from the RRE. If random noise is detected within the RRE of a duration greater than about say 25% of the total RRE then the RRE signal to noise may be compromised, then the RRE may be marked as non-RRE.

### 4.2.2.3 Event Detection 180

The prolonged duration of reliable breathing or snoring signals contained in each RRE make it a good candidate for determination of certain conditions. Therefore, process 180 may be applied to frames marked as a RRE to detect respiratory events such as events of Sleep Disordered Breathing (SDB).

Optionally, to ensure that sufficient data is available for analysis, process 180 may be initiated upon confirmation of existence of RRE, for example by considering a detection time threshold with the time of a suitable RRE. Thus, a total duration time of an RRE may be evaluated to determine if it exceeds a predetermined RRE threshold time, such as about 2 minutes. For example, if the RRE has 1,000 duration frame files, each frame being 0.25s in duration, then the amount of detection time in each RRE will be 4.167 minutes. Depending on the detection threshold time, the RRE may be evaluated for detection of events therein.

If the RRE detection threshold has been met by at least one RRE, then process 180 may proceed to analyze the audio signal (such as with an analysis of its frequency components) of each confirmed RRE to determine if the characteristics associated with certain events are present. For example, the presence of an apnea snoring may be determined based upon detecting a period of silence such as within a range of time between the laboured breathing or snoring. See, e.g., Fig. 6B; a period of snoring of say 60s is followed by one or a number of periods of silence of say 10-60s with brief periods of snoring of say 15s-60s in between followed by a continuous period of snoring.

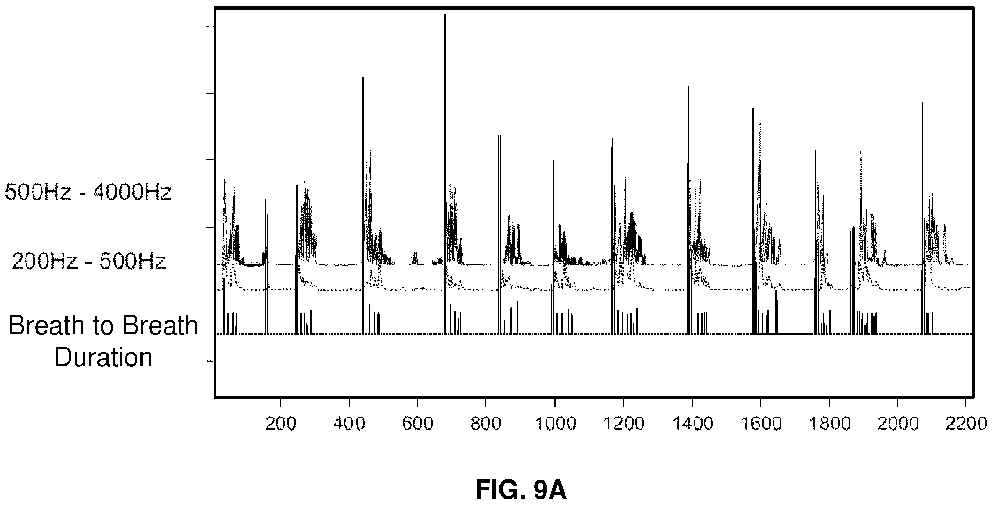

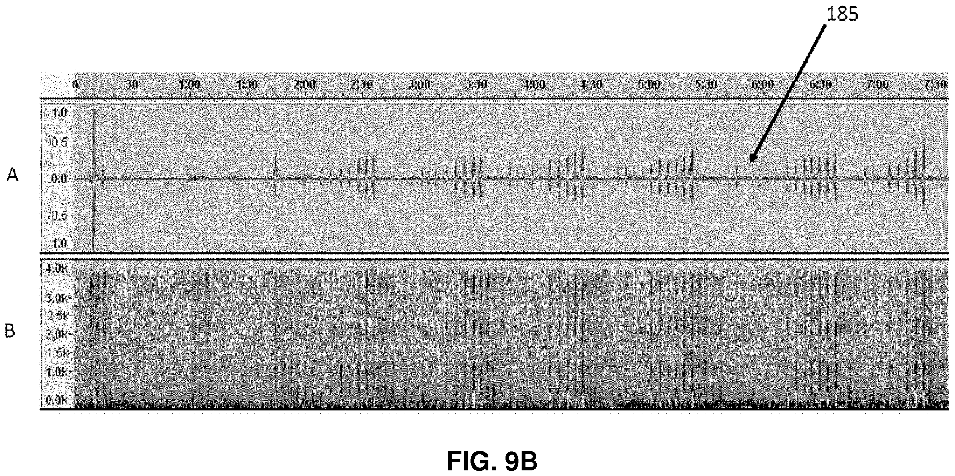

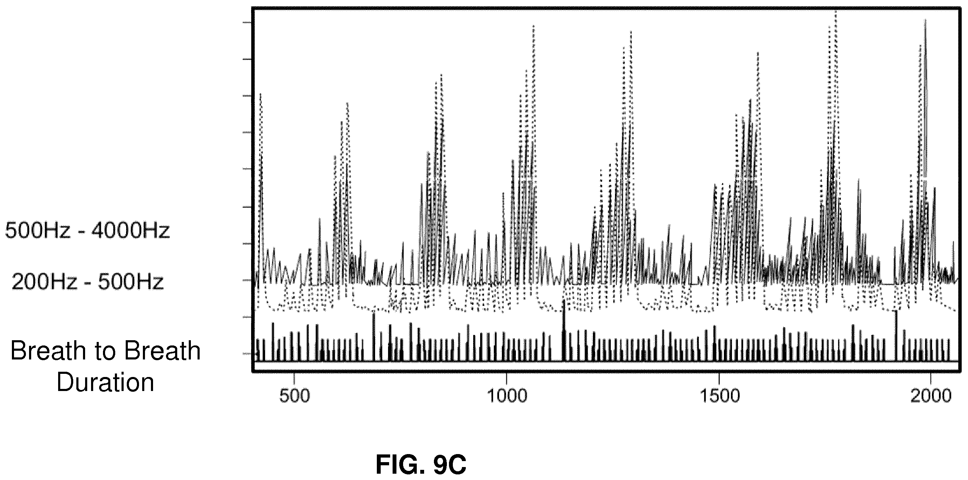

The presence of an apnea may also be determined based upon detecting a period of silence such as within a range of time between quiet breathing. See, e.g., Figs. 6I and 9A; this is similar to the above, with detectable breathing instead of snoring. Optionally, such apnea events may be determined by detection of a post-apnea recovery breath such as that illustrated in Fig. 6G. Such events may be detected by analyzing recovery breathing audio level, rate and/or frequency after a detected apnea event or period of silence. Recovery breaths have an acoustic audio level, rate and/or frequency fingerprint. This fingerprint is a strong indicator of the presence of a preceding apnea event. As a further example, signal 12 of FIG. 9B does not contain an apnea; however, the presence of some type of modulated breathing (i.e. CSR) can be determined based upon a repeating series of crescendos, each marked as a crescendo 185 within signal 12 in FIG. 9B. Although not illustrated, other conditions, like hypopnea, may also be detected. Modulated breathing has an amplitude-modulated acoustic fingerprint. This fingerprint is a strong indicator of the presence of a modulated breathing event. In some cases, snoring may be determined by any of the methodologies described in International Patent Application No. PCT/US2014/059311, filed October 6, 2014, the entire disclosure of which is incorporated herein by reference.

Process 180 may then generate indicators of such events, for example by quantifying them such as by time and/or count. For example, an Apnea/Hypopnea count may be generated.

In some versions, respiration rates for each RRE, such as a rate detected from breathing or detected from snoring in the RRE, may be determined and variations between the respiration rates, such as between any two or more RREs may be calculated. Sleep parameters may be determined such as sleep time, snore time, AHI and a snore score (e.g., a ration of overall snore time to overall sleep time) such as based on the determined RRE.

Still other indictors may be based upon events of the RREs.

### 4.2.2.4 Output Reporting 190

The screening device 20 may further include a reporting process 190 based on events detection process and RRE detection process. In some embodiments of the present technology, process 190 may report (e.g., output on a display of screening device or transmit information from screening device 20) detected indicators to help the user. The indicia may include a summary of the determination made in process 180 together with any SDB indicator data, such as one or more of the scores described above. The summary may be on a session by session basis so as to allow display of a single session or multiple sessions over time.

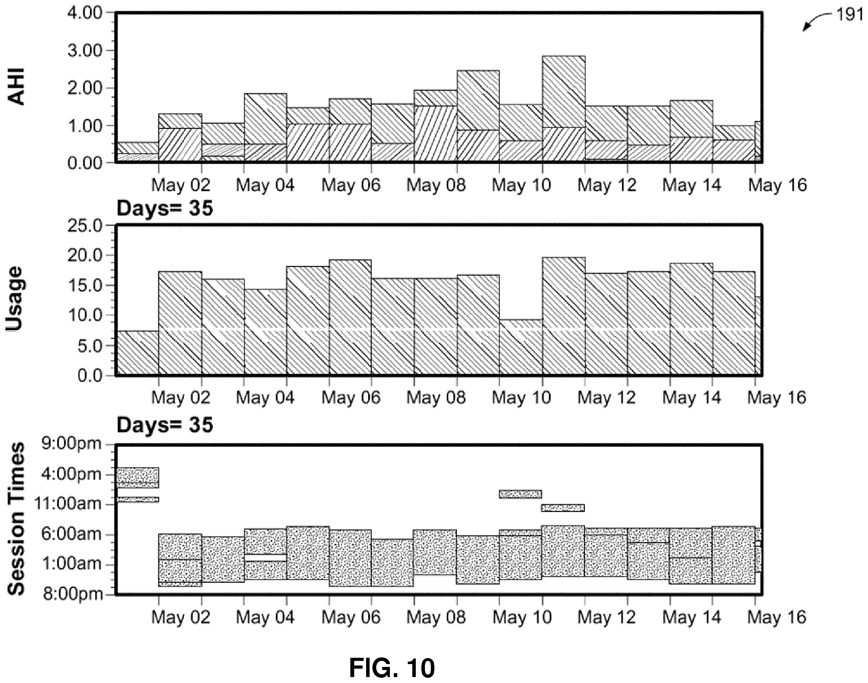

For example, as illustrated in Fig. 10, a report 191 of diagnostic details may be generated and output. The report 191 may be presented on a display screen of device 20. Example types of indicia that may be included in such a report are:
(A) the presence of any SDB events, such as apnea, hypopnea, snore and CSR;
(B) measurement indices and statistics (e.g., event counts such as an AHI; or period based - such as sleep score)
(C) historical data in tabular and graphical format, such as usage time, session time, etc. ;
(D) snoring time; and
(E) any associated details, such as the presence of any fault conditions that occurred during the detection period or absence of RREs.
Based upon these indicia, user 10 is may gain an awareness of his/her sleep breathing state to be able to consider further diagnosis or treatment. For example, a suggestion of a need for further testing for an SDB condition may be presented to the user together with information on SDB treatment options.

In other forms of the present technology, the indicia from process 190 may optionally also be output one or more third parties such as via wired or wireless communications link with the screening device 20. The indicia may, for example, be sent together with a portion of input signal 12 via transmitter of the device 20 to a database for alternative of subsequent analysis, or other use. Alternatively, portions of the indicia may be concurrently sent to the user and various databases with signal 12 for subsequent analysis or use.

### 4.3 GLOSSARY

For the purposes of the present technology disclosure, in certain forms of the present technology, one or more of the following definitions may apply. In other forms of the present technology, alternative definitions may apply.

### 4.3.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Continuous Positive Airway Pressure (CPAP) therapy*: CPAP therapy will be taken to mean the application of a supply of air to an entrance to the airways at a pressure that is continuously positive with respect to atmosphere. The pressure may be approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Patient*: A person, whether or not they are suffering from a respiratory disease.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

### 4.3.2 Aspects of the respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: Breathing effort will be said to be the work done by a spontaneously breathing person attempting to breathe.

*Expiratory portion of a breathing cycle*: The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened*: Having a rise followed by a relatively flat portion, followed by a fall.
(ii) *M-shaped*: Having two local peaks, one at the leading edge, and one at the trailing edge, and a relatively flat portion between the two peaks.
(iii) *Chair-shaped*: Having a single local peak, the peak being at the leading edge, followed by a relatively flat portion.
(iv) *Reverse-chair shaped*: Having a relatively flat portion followed by single local peak, the peak being at the trailing edge.

*Hypopnea*: Preferably, a hypopnea will be taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal flow rate.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow* rate *(Qpeak)*: The maximum value of flow rate during the inspiratory portion of the respiratory flow waveform.

*Respiratory flow* rate, *airflow* rate, *patient airflow* rate, *respiratory airflow* rate *(Qr)*: These synonymous terms may be understood to refer to the RPT device's estimate of respiratory airflow rate, as opposed to "true respiratory flow" or "true respiratory airflow", which is the actual respiratory flow rate experienced by the patient, usually expressed in litres per minute.

*Tidal volume (Vt)*: The volume of air inhaled or exhaled during normal breathing, when extra effort is not applied.

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of the inspiratory portion of one respiratory flow rate waveform and the start of the inspiratory portion of the following respiratory flow rate waveform.

*Typical recent ventilation*: The value of ventilation around which recent values over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the level of flow increases only slightly or may even decrease as the pressure difference across the upper airway increases (Starling resistor behaviour).

*Ventilation* (*Vent*): A measure of the total amount of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.4 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the present technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.
The technology has been described with particular emphasis on the detection of sleep disorder breathing events, it has to be noted that the same principals are applicable to the detection of any of the types of sound described in the included table, including coughing or other sounds.

## Claims

1. A computer program comprising instructions which, when the program is executed by a processor (40), cause the processor to carry out a method for detecting an event of sleep disordered breathing of a user, the method comprising:
receiving, in the processor, an input audio signal representing sounds of the user during a period of sleep (110);
determining, in the processor, from the received input audio signal, at least one reliable respiration epoch - RRE, the at least one determined RRE including at least a period associated with one or both of audible breathing and snoring;
detecting, in the processor and based on data in the at least one determined RRE, presence of a sleep disordered breathing event in the at least one determined RRE (180), the sleep disordered breathing event being at least one of snoring, an apnea event, a hypopnea event, and a modulated breathing event;
analyzing respiratory rate of a breathing signal determined from the input audio signal to detect recovery breathing after a detected quiet period to confirm an apnea event; and
outputting, from the processor, an indicator of the detected sleep disordered breathing event (190).

2. The computer program of claim 1, the analyzing to detect the recovery breathing further comprises analysing audio level of the received input audio signal.

3. The computer program of any one of claims 1 to 2, wherein the analyzing to detect the recovery breathing comprises classifying an event as recovery breathing.

4. The computer program of any one of claims 1 to 3, wherein the analyzing to detect the recovery breathing comprises implementing frame filtering that groups a plurality of frequency components of the input audio signal into categories of audio frequency bands based upon signal energy in the audio frequency bands,
and optionally, wherein the method further comprises summating audio signal level for each of the audio frequency bands.

5. The computer program of any one of claims 1 to 4, wherein a signal type representing the detected recovery breathing is **characterized by** frequency components in a frequency band of 100 Hertz to 4000 Hertz.

6. The computer program of any one of claims 1 to 5, wherein the analyzing to detect the recovery breathing comprises detecting an audio level amplitude that is higher than an average amplitude of breathing, wherein the detected audio level amplitude is at least twice as high as the average amplitude of breathing signal.

7. The computer program of any one of claims 1 to 6, wherein:
(a) the analyzing to detect the recovery breathing comprises detecting an audio level amplitude that is higher than an average amplitude of snoring signal; and/or
(b) the analyzing to detect the recovery breathing comprises detecting a breathing rate that is higher than an average breathing rate,
and optionally, wherein the detected breathing rate is in a range of 20 beats per minute to 30 beats per minute.

8. The computer program of any one of claims 1 to 7, wherein:
(a) detecting at least one recovery breath of the recovery breathing comprises detecting an audio frequency that is higher than an average frequency of snoring; and/or
(b) detecting at least one recovery breath of the recovery breathing comprises detecting an audio frequency that is higher than an average frequency of breathing.

9. The computer program of any one of claims 1 to 8, wherein the analyzing to detect the recovery breathing comprises providing a probability value for a signal type representing at least one recovery breath of the recovery breathing,
and optionally, wherein the probability value is a highest probability value of a plurality of determined probability values for a group of different signal types.

10. The computer program of any one of claims 1 to 9, wherein at least one recovery breath of the recovery breathing is associated with an end of an apnea; and
wherein, preferably, at least one recovery breath of the recovery breathing is a gasp with snoring.

11. The computer program of any one of claims 1 to 10, further comprising, detecting, in the processor and based on the detected recovery breathing, a presence of apnea by detecting a period of silence preceding the detected recovery breathing.

12. A computer-readable medium having stored thereon the computer program of any one of claims 1 to 11.

13. Apparatus (20) for detecting an event of sleep disordered breathing of a user, the apparatus comprising:
a sound sensor (30) configured to sense sound proximate to the sensor and generate an audio signal representing the sensed sound; and
a processor (40) configured to:
receive an input audio signal representing sounds of the user during a period of sleep (110);
determine, from the received input audio signal, at least one reliable respiration epoch - RRE, the epoch including at least a period associated with one or both of audible breathing and snoring (160);
detect, based on data in the at least one determined RRE, presence of a sleep disordered breathing event in the at least one determined RRE (180), the sleep disordered breathing event being at least one of snoring, an apnea event, a hypopnea event, and a modulated breathing event;
analyze respiratory rate of a breathing signal determined from the received input audio signal to detect recovery breathing after a detected quiet period to confirm an apnea event; and
output an indicator of the detected sleep disordered breathing event (190).

14. The apparatus (20) of claim 13 wherein: (a) the processor (40) is configured to perform the computer program of any one of claims 1 to 11, and/or (b) the apparatus (20) comprises the computer-readable medium of claim 12.

## Patentansprüche

1. Computerprogramm aufweisend Befehle, die bei Ausführung des Programms durch einen Prozessor (40) bewirken, dass der Prozessor ein Verfahren zum Detektieren eines schlafbezogenen Atemstörungsereignisses eines Benutzers durchführt, wobei das Verfahren aufweist:
im Prozessor erfolgendes Empfangen eines Eingangsaudiosignals, das Geräusche des Benutzers während einer Schlafperiode darstellt (110);
im Prozessor anhand des empfangenen Eingangsaudiosignals erfolgendes Bestimmen mindestens einer zuverlässigen Atemepoche (reliable respiration epoch - RRE), wobei die mindestens eine bestimmte RRE mindestens eine Periode aufweist, die hörbarer Atmung und/oder Schnarchen zugeordnet ist;
im Prozessor und auf der Grundlage von Daten in der mindestens einen bestimmten RRE erfolgendes Detektieren des Vorliegens eines schlafbezogenen Atemstörungsereignisses in der mindestens einen bestimmten RRE (180), wobei das schlafbezogene Atemstörungsereignis Schnarchen, ein Apnoeereignis, ein Hypopnoeereignis und/oder ein moduliertes Atemereignis ist;
Analysieren der Atemfrequenz eines anhand des Eingangsaudiosignals bestimmten Atmungssignals, um Erholungsatmung nach einer detektierten Stilleperiode zu detektieren, um ein Apnoeereignis zu bestätigen; und
vom Prozessor erfolgendes Ausgeben eines Indikators des detektierten schlafbezogenen Atemstörungsereignisses (190).

2. Computerprogramm nach Anspruch 1, wobei das Analysieren, um die Erholungsatmung zu detektieren, ferner aufweist: Analysieren eines Audiopegels des empfangenen Eingangsaudiosignals.

3. Computerprogramm nach einem der Ansprüche 1 bis 2, wobei das Analysieren, um die Erholungsatmung zu detektieren, aufweist: Klassifizieren eines Ereignisses als Erholungsatmung.

4. Computerprogramm nach einem der Ansprüche 1 bis 3, wobei das Analysieren, um die Erholungsatmung zu detektieren, aufweist: Implementieren von Rahmenfilterung, die mehrere Frequenzkomponenten des Eingangsaudiosignals in Kategorien von Audiofrequenzbändern auf der Grundlage der Signalenergie in den Audiofrequenzbändern gruppiert,
und wobei optional das Verfahren ferner aufweist: Summieren des Audiosignalpegels für jedes der Audiofrequenzbänder.

5. Computerprogramm nach einem der Ansprüche 1 bis 4, wobei eine die detektierte Erholungsatmung darstellende Signalart durch Frequenzkomponenten in einem Frequenzband von 100 Hertz bis 4000 Hertz gekennzeichnet ist.

6. Computerprogramm nach einem der Ansprüche 1 bis 5, wobei das Analysieren, um die Erholungsatmung zu detektieren, aufweist: Detektieren einer Audiopegelamplitude, die höher ist als eine durchschnittliche Atmungsamplitude, wobei die detektierte Audiopegelamplitude mindestens doppelt so hoch ist wie die durchschnittliche Atmungssignalamplitude.

7. Computerprogramm nach einem der Ansprüche 1 bis 6, wobei:
(a) das Analysieren, um die Erholungsatmung zu detektieren, aufweist: Detektieren einer Audiopegelamplitude, die höher ist als eine durchschnittliche Schnarchsignalamplitude; und/oder
(b) das Analysieren, um die Erholungsatmung zu detektieren, aufweist: Detektieren einer Atemfrequenz, die höher ist als eine durchschnittliche Atemfrequenz,
und wobei optional die detektierte Atemfrequenz in einem Bereich von 20 Schlägen pro Minute bis 30 Schlägen pro Minute liegt.

8. Computerprogramm nach einem der Ansprüche 1 bis 7, wobei:
(a) das Detektieren mindestens eines Erholungsatemzugs der Erholungsatmung aufweist: Detektieren einer Audiofrequenz, die höher ist als eine durchschnittliche Schnarchfrequenz; und/oder
(b) das Detektieren mindestens eines Erholungsatemzugs der Erholungsatmung aufweist: Detektieren einer Audiofrequenz, die höher ist als eine durchschnittliche Atemfrequenz.

9. Computerprogramm nach einem der Ansprüche 1 bis 8, wobei das Analysieren, um die Erholungsatmung zu detektieren, aufweist: Bereitstellen eines Wahrscheinlichkeitswerts für eine Signalart, die mindestens einen Erholungsatemzug der Erholungsatmung darstellt,
und wobei optional der Wahrscheinlichkeitswert ein höchster Wahrscheinlichkeitswert mehrerer bestimmter Wahrscheinlichkeitswerte für eine Gruppe unterschiedlicher Signalarten ist.

10. Computerprogramm nach einem der Ansprüche 1 bis 9, wobei mindestens ein Erholungsatemzug der Erholungsatmung einem Ende einer Apnoe zugeordnet ist; und wobei vorzugsweise mindestens ein Erholungsatemzug der Erholungsatmung ein Schnappatemzug mit Schnarchen ist.

11. Computerprogramm nach einem der Ansprüche 1 bis 10, das ferner aufweist: im Prozessor und auf der Grundlage der detektierten Erholungsatmung erfolgendes Detektieren eines Vorliegens von Apnoe durch Detektieren einer Stilleperiode, die der detektierten Erholungsatmung vorausgeht.

12. Computerlesbares Medium mit dem darauf gespeicherten Computerprogramm nach einem der Ansprüche 1 bis 11.

13. Vorrichtung (20) zum Detektieren eines schlafbezogenen Atemstörungsereignisses eines Benutzers, wobei die Vorrichtung aufweist:
einen Geräuschsensor (30), der so konfiguriert ist, dass er Geräusche in der Nähe des Sensors erfasst und ein die erfassten Geräusche darstellendes Audiosignal erzeugt; und
einen Prozessor (40), der so konfiguriert ist, dass er:
ein Eingangsaudiosignal empfängt, das Geräusche des Benutzers während einer Schlafperiode darstellt (110);
anhand des empfangenen Audiosignals mindestens eine zuverlässige Atemepoche (reliable respiration epoch - RRE) bestimmt, wobei die Epoche mindestens eine Periode aufweist, die hörbarer Atmung und/oder Schnarchen zugeordnet ist (160);
auf der Grundlage von Daten in der mindestens einen bestimmten RRE das Vorliegen eines schlafbezogenen Atemstörungsereignisses in der mindestens einen bestimmten RRE detektiert (180), wobei das schlafbezogene Atemstörungsereignis Schnarchen, ein Apnoeereignis, ein Hypopnoeereignis und/oder ein moduliertes Atemereignis ist;
die Atemfrequenz eines anhand des empfangenen Eingangsaudiosignals bestimmten Atmungssignals analysiert, um Erholungsatmung nach einer detektierten Stilleperiode zu detektieren, um ein Apnoeereignis zu bestätigen; und
einen Indikator des detektierten schlafbezogenen Atemstörungsereignisses ausgibt (190).

14. Vorrichtung (20) nach Anspruch 13, wobei (a) der Prozessor (40) so konfiguriert ist, dass er das Computerprogramm nach einem der Ansprüche 1 bis 11 durchführt, und/oder (b) die Vorrichtung (20) das computerlesbare Medium nach Anspruch 12 aufweist.

## Revendications

1. Un programme d'ordinateur comprenant des instructions qui, lorsque le programme est exécuté par un processeur (40), amènent le processeur à mettre en œuvre un procédé de détection d'un événement de troubles respiratoires liés au sommeil d'un utilisateur, le procédé comprenant:
recevoir, dans le processeur, un signal audio d'entrée représentant des sons de l'utilisateur pendant une période de sommeil (110) ;
déterminer, dans le processeur, à partir du signal audio d'entrée reçu, au moins un intervalle de respiration fiable, dit IRF, l'au moins un IRF déterminé comprenant au moins une période associée à l'un ou aux deux parmi une respiration audible et un ronflement ;
détecter, dans le processeur et sur la base de données de l'au moins un IRF déterminé, la présence d'un événement de troubles respiratoires liés au sommeil pendant l'au moins un IRF déterminé (180), l'événement de troubles respiratoires liés au sommeil étant au moins l'un d'un ronflement, d'un événement d'apnée, d'un événement d'hypopnée et d'un événement de respiration modulée ;
analyser un rythme respiratoire d'un signal de respiration déterminé à partir du signal audio d'entrée pour détecter une reprise de respiration après une période de silence détectée pour confirmer un événement d'apnée ; et
délivrer, par le processeur, un indicateur de l'événement de respiration de trouble du sommeil détecté (190).

2. Le programme d'ordinateur selon la revendication 1, dans lequel l'analyse pour détecter la reprise de respiration consiste en outre à analyser un niveau audio du signal audio d'entrée reçu.

3. Le programme d'ordinateur selon l'une quelconque des revendications 1 et 2, dans lequel l'analyse pour détecter la reprise de respiration consiste à classifier un événement en tant que reprise de respiration.

4. Le programme d'ordinateur selon l'une quelconque des revendications 1 à 3, dans lequel l'analyse pour détecter la reprise de respiration consiste à mettre en œuvre un filtrage de trame qui regroupe une pluralité de composantes de fréquences du signal audio d'entrée en catégories de bandes de fréquences audio sur la base d'une énergie de signal des bandes de fréquences audio,
et optionnellement, dans lequel le procédé comprend en outre sommer un niveau de signal audio de chacune des bandes de fréquences audio.

5. Le programme d'ordinateur selon l'une quelconque des revendications 1 à 4, dans lequel un type de signal représentant la reprise de respiration détectée est **caractérisé par** des composantes de fréquences dans un intervalle de fréquences de 100 Hertz à 4000 Hertz.

6. Le programme d'ordinateur selon l'une quelconque des revendications 1 à 5, dans lequel l'analyse pour détecter la reprise de respiration consiste à détecter une amplitude de niveau audio qui est supérieure à une amplitude moyenne de respiration, dans lequel l'amplitude de niveau audio détectée est au moins deux fois aussi élevée que l'amplitude moyenne de signal de respiration.

7. Le programme d'ordinateur selon l'une quelconque des revendications 1 à 6, dans lequel :
(a) l'analyse pour détecter la reprise de respiration consiste à détecter une amplitude de niveau audio qui est supérieure à une amplitude moyenne de signal de ronflement ; et/ou
(b) l'analyse pour détecter la reprise de respiration consiste à détecter un rythme de respiration qui est supérieur à un rythme de respiration moyen,
et optionnellement, dans lequel le rythme de respiration détecté s'inscrit dans une plage de 20 battements par minute à 30 battements par minute.

8. Le programme d'ordinateur selon l'une quelconque des revendications 1 à 7, dans lequel :
(a) la détection d'au moins une respiration de reprise de la reprise de respiration comprend détecter une fréquence audio qui est supérieure à une fréquence moyenne de ronflement ; et/ou
(b) la détection d'au moins une respiration de reprise de la reprise de respiration comprend détecter une fréquence audio qui est supérieure à une fréquence moyenne de respiration.

9. Le programme d'ordinateur selon l'une quelconque des revendications 1 à 8, dans lequel l'analyse pour détecter la reprise de respiration consiste à fournir une valeur de probabilité pour un type de signal représentant au moins une respiration de reprise de la reprise de respiration,
et optionnellement, dans lequel la valeur de probabilité est une valeur de probabilité la plus élevée d'une pluralité de valeurs de probabilité déterminées d'un groupe de différents types de signaux.

10. Le programme d'ordinateur selon l'une quelconque des revendications 1 à 9, dans lequel au moins une respiration de reprise de la reprise de respiration est associée à une fin d'une apnée ; et
dans lequel, de préférence, au moins une respiration de reprise de la reprise de respiration est un halètement avec ronflement.

11. Le programme d'ordinateur selon l'une quelconque des revendications 1 à 10, comprenant en outre détecter, dans le processeur et sur la base de la reprise de respiration détectée, une présence d'apnée par une détection d'une période de silence précédant la reprise de respiration détectée.

12. Un support lisible par ordinateur comportant sur lequel est enregistré le programme d'ordinateur selon l'une quelconque des revendications 1 à 11.

13. Appareil (20) destiné à détecter un événement de respiration de trouble du sommeil d'un utilisateur, l'appareil comprenant :
un capteur de son (30) configuré pour détecter un son à proximité du capteur et pour générer un signal audio représentant le son détecté ; et
un processeur (40) configuré pour :
recevoir un signal audio d'entrée représentant des sons de l'utilisateur pendant une période de sommeil (110) ;
déterminer, à partir du signal audio d'entrée reçu, au moins un intervalle de respiration fiable, dit IRF, l'intervalle comprenant au moins une période associée à l'un ou aux deux parmi une respiration audible et un ronflement (160) ;
détecter, sur la base de données de l'au moins un IRF déterminé, la présence d'un événement de respiration de trouble du sommeil sur l'au moins un IRF déterminé (180), l'événement de respiration de trouble du sommeil étant au moins l'un d'un ronflement, d'un événement d'apnée, d'un événement d'hypopnée et d'un événement de respiration modulée ;
analyser un rythme respiratoire d'un signal de respiration déterminé à partir du signal audio d'entrée pour détecter une reprise de respiration après une période de silence détectée pour confirmer un événement d'apnée ; et
délivrer un indicateur de l'événement de respiration de trouble du sommeil détecté (190).

14. L'appareil (20) selon la revendication 13, dans lequel : (a) le processeur (40) est configuré pour mettre en œuvre le programme d'ordinateur selon l'une quelconque des revendications 1 à 11, et/ou (b) l'appareil (20) comprend le support lisible par ordinateur selon la revendication 12.
